# EUROPEAN PATENT APPLICATION

(11) **EP 4 700 044 A1**
(43) Date of publication of application: **25.02.2026**
(21) Application number: 24778266.7
(22) Date of filing: 29.03.2024
(51) Int. Cl.: C07K 14/50, C12N 15/12

(54) **PROTEIN ANALOGUE AND USE THEREOF**

(30) Priority: 31.03.2023 CN 202310338538
(71) Applicant: Shanghai Duomirui Biotechnology., Ltd., Shanghai 201419 (CN)
(72) Inventor: FENG, Jun, Shanghai 201419 (CN); WANG, Yapeng, Shanghai 201419 (CN); LU, Jianguang, Shanghai 201419 (CN); HUANG, Zongqing, Shanghai 201419 (CN); DONG, Yuanzhen, Shanghai 201419 (CN); ZHANG, Ying, Shanghai 201419 (CN)
(74) Representative: Michalski Hüttermann & Partner Patentanwälte mbB
(86) International application number: PCT/CN2024/084988
(87) International publication number: WO 2024/199481

(57) **Abstract**

A modified protein. The modified protein comprises a protein moiety and a modified moiety. An optional PAS is linked to the N-terminus of the protein moiety, the modified moiety is directly or by means of a linker linked to a cysteine residue in the protein moiety or in the optional PAS, and the cysteine residue is naturally occurring or introduced by mutation. The original activity of the modified protein is retained, and the half-life of the modified protein in vivo is also significantly improved. The modified protein involves a simple preparation process and is suitable for the development of a series of drugs for treating non-alcoholic steatohepatitis, type 2 diabetes, hyperlipidemia, atherosclerosis and the like.

## Description

### Technical field

The present invention relates to the field of biomedicine. In particular, the present invention relates to a modified protein, especially a fatty acid-modified protein, and uses thereof in the treatment of a disease.

### Background

Nonalcoholic steatohepatitis (NASH) is an inflammatory subtype of nonalcoholic fatty liver disease (NAFLD), characterized by hepatocellular inflammation and fibrosis. Some patients may progress to cirrhosis and liver cancer. Epidemiological studies have reported that 3-6% of the adult population in the United States suffers from NASH, and it has become one of the leading causes of liver transplantation. The number of NASH patients in China is estimated to reach 30 million. NASH is a chronic disease with diverse etiologies and complex pathological processes, accompanied by various metabolic complications, which makes the treatment complicated. So far, there is no NASH treatment drug that has passed rigorous randomized controlled clinical trials, thus approved and released.

Fibroblast Growth Factor 21 (FGF21) is a stress-induced hormone that regulates energy balance and glucose-lipid metabolism, belonging to the endocrine FGFs. Preclinical studies have shown that FGF21 has physiological and pharmacological activities, such as reducing body weight, improving dyslipidemia, increasing insulin sensitivity, and reducing hepatic steatosis and fibrosis, demonstrating great potential in the treatment of NASH.

Although FGF21 has potential therapeutic effects on NASH, its short half-life and poor stability require frequent injections, which seriously affect patient compliance. Therefore, biopharmaceutical companies have adopted various technical means to modify it to improve its druggability. LY-2405319 developed by Lilly enhances its stability by truncating the N-terminal HPIP sequence and introducing cysteine mutations to form additional disulfide bonds (L118C, A134C), and a S167A mutation was introduced to eliminate O-glycosylation produced by yeast expression. The once-weekly subcutaneous injection long-acting FGF21 BMS-986036 (pegbelfermin) developed by Bristol-Myers Squibb is obtained by inserting the non-natural amino acid pAcF (Q108pAcF) and modifying it with PEG containing alkoxy-amino groups through bioorthogonal reaction, which prolongs the half-life while increasing solubility and stability. In AKR-001 developed by Amgen, three point mutations (L98R, P171G, and A180E) were conducted on human FGF21 which was fused with the IgG1 Fc fragment for expression, thereby achieving the goals of reducing aggregation, decreasing *in vivo* enzymatic hydrolysis, and prolonging the half-life. Pfizer modifies FGF21 through two methods: one is to connect FGF21 to a specific site of the framework antibody CVX-2000 Fab (PF-05231023), giving it pharmacokinetic properties similar to an antibody; the other is to mutate the potential glycosylation sites of FGF21 expressed in CHO cells and fuse it with the Fc fragment (PF-06645849).

However, the modification methods for these FGF21 analogs still have many problems. For example, PEG cannot be metabolized *in vivo,* thus causing toxicity concerns, and Fc fusion proteins have problems, such as stability and glycosylation. In recent years, in addition to PEG modification and Fc fusion technology, several other technologies have been used for long-acting modification of peptide and protein drugs, such as fusion with human serum albumin, fatty acid modification, and modification with PEG-like repeated polypeptide sequences. Human serum albumin (HSA) is the most abundant protein in human plasma, with a molecular weight of approximately 67 kDa and a half-life in human plasma as long as 19 days. Similar to the antibody Fc segment, HSA also has a property of binding to the neonatal receptor (FcRn) in a pH-dependent manner to escape the lysosomal degradation pathway, thus having a relatively long half-life. Therefore, the fusion expression with HSA is an ideal long-acting method for biopharmaceuticals. For example, the *in vivo* half-life of albiglutide is as long as 4-7 days. However, HSA has a large molecular weight, and the fusion expression with a functional polypeptide or protein can easily lead to a decrease in biological activities and may limit the biodistribution of the drug. As the shortcomings of PEG modification are gradually exposed, many technologies playing a role similar to PEG have gradually become alternative means to optimize the pharmacokinetic properties of biopharmaceuticals. XL Protein biotechnology company (German) discovered that polypeptide chains consisting of three amino acids: Pro, Ala, and Ser (PAS) have PEG-like characteristics, such as high hydrophilicity and high water-solubility, and when fused with growth hormone, interferon, and Fab fragments, they can significantly increase *in vivo* activity and half-life. Compared with methods requiring *in vitro* modification such as PEG modification, PAS modification has an advantage of being expressible as a fusion protein in *Escherichia coli.*

### Summary of the invention

The purpose of the present invention is to provide an active protein medicament, such as a mutant of FGF21, which can not only retain the original activity but also have an improved half-life.

In the first aspect, the present invention provides a modified protein, wherein the modified protein includes a protein moiety and a modifying moiety, the protein moiety is optionally linked to PAS at the N-terminus, the modifying moiety is connected directly or via a connector to a cysteine residue in the protein moiety or a cysteine residue in the PAS, and the cysteine residue is either naturally occurring or introduced through mutation.

In a preferred embodiment, the mutation is a substitution or insertion; preferably, a substitution.

In a preferred embodiment, the protein is a cell growth factor, growth hormone, interferon, enzyme, antibody, or an active fragment thereof.

In a preferred embodiment, the cell growth factor is epidermal fibroblast growth factor, platelet-derived fibroblast growth factor, fibroblast growth factor, insulin-like fibroblast growth factor, nerve fibroblast growth factor, interleukin-like fibroblast growth factor, erythropoietin, or colony-stimulating factor.

In a specific embodiment, the fibroblast growth factor is fibroblast growth factor 21.

In a preferred embodiment, the amino acid sequence of the fibroblast growth factor 21 is shown in SEQ ID NO: 1 or 2.

In a preferred embodiment, the amino acid sequence of the fibroblast growth factor 21 is shown in SEQ ID NO: 1 or 2, and cysteine residues are introduced therein through mutation.

In a preferred embodiment, the amino acid sequence of the fibroblast growth factor 21 is shown in SEQ ID NO: 1 or 2, and Q28, E30, D38, D46, K122 or H125 is mutated to a cysteine residue.

In a preferred embodiment, the amino acid sequence of the fibroblast growth factor 21 is shown in any one of SEQ ID NO: 4-9.

In a preferred embodiment, the amino acid sequence of the fibroblast growth factor 21 is shown in any one of SEQ ID NO: 5-9.

In a preferred embodiment, the modifying moiety can extend the *in vivo* half-life of the protein moiety.

In a preferred embodiment, the modifying moiety can specifically or non-specifically bind to a protein in plasma.

In a preferred embodiment, the protein in plasma has a long half-life; for example, the half-life of the protein in plasma is 10 days or more, preferably 15 days or more, and more preferably 19 days or more.

In a preferred embodiment, the protein in plasma is human serum albumin.

In a specific embodiment, the modifying moiety is an albumin-binding agent.

In a preferred embodiment, the albumin-binding agent is a fatty acid.

In a preferred embodiment, the fatty acid is a C18-22 fatty acid.

In a preferred embodiment, the EC₅₀ value of the binding affinity between the fatty acid and human serum albumin is preferably less than about 10 µM; more preferably less than about 1 µM.

In a specific embodiment, the structure of the albumin-binding agent is shown in the following formula:

R₁-(X₁)ₘ₋(X₂)ₙ-K(ε-R₂);

wherein K is a lysine;
R₁ is a substituted or unsubstituted C₁₋₂₀ acyl;
m is an integer from 0 to 5;
n is an integer from 0 to 5;
X₁ and X₂ are independently selected from the following group: alanine (Ala), D-alanine (D-Ala), β-alanine (β-Ala), 4-aminobutyric acid (GABA), 2-aminoisobutyric acid (Aib), 2-aminobutyric acid (Abu), arginine (Arg), aspartic acid (Asp), asparagine (Asn), cysteine (Cys), glutamic acid (Glu), D-glutamic acid (D-Glu), γ-glutamic acid (γ-Glu), glutamine (Gln), glycine (Gly), histidine (His), isoleucine (Ile), leucine (Leu), lysine (Lys), proline (Pro), phenylalanine (Phe), serine (Ser), tyrosine (Tyr), threonine (Thr), tryptophan (Trp), valine (Val), methionine (Met), tranexamic acid (Trx), AEEA, PEG;
R₂ is selected from a halogen-substituted C₁₋₆ acyl or H.

In a preferred embodiment, R₁ is selected from: a heptanoyl, methylheptanoyl, octanoyl, methyloctanoyl, nonanoyl, methylnonanoyl, decanoyl, methyldecanoyl, lauroyl, myristoyl, palmitoyl, stearoyl, 17-carboxyheptadecanoyl, 15-carboxypentadecanoyl, 13-carboxytridecanoyl, 11-carboxyundecanoyl.

In a preferred embodiment, R₁ is selected from: a lauroyl, myristoyl, palmitoyl, stearoyl, 17-carboxyheptadecanoyl, 19-carboxynonadecanoyl.

In a preferred embodiment, m is an integer from 1 to 3.

In a preferred embodiment, n is an integer from 1 to 3.

In a preferred embodiment, X₁ and X₂ are independently selected from the group consisting of 4-aminobutyric acid (GABA), 2-aminoisobutyric acid (Aib), D-alanine (D-Ala), β-alanine (β-Ala), aspartic acid (Asp), cysteine (Cys), glutamic acid (Glu), γ-glutamic acid (γ-Glu), glycine (Gly), serine (Ser), and tyrosine (Tyr).

In a preferred embodiment, X₁ and X₂ are independently selected from the group consisting of glutamic acid (Glu), γ-glutamic acid (γ-Glu), and tyrosine (Tyr).

In a preferred embodiment, R₂ is selected from a halogen-substituted C₁₋₃ acyl or H.

In a preferred embodiment, R₂ is selected from an iodoacetyl, bromoacetyl, or H; more preferably bromoacetyl or H.

In a specific embodiment, the albumin-binding agent is selected from the group consisting of:

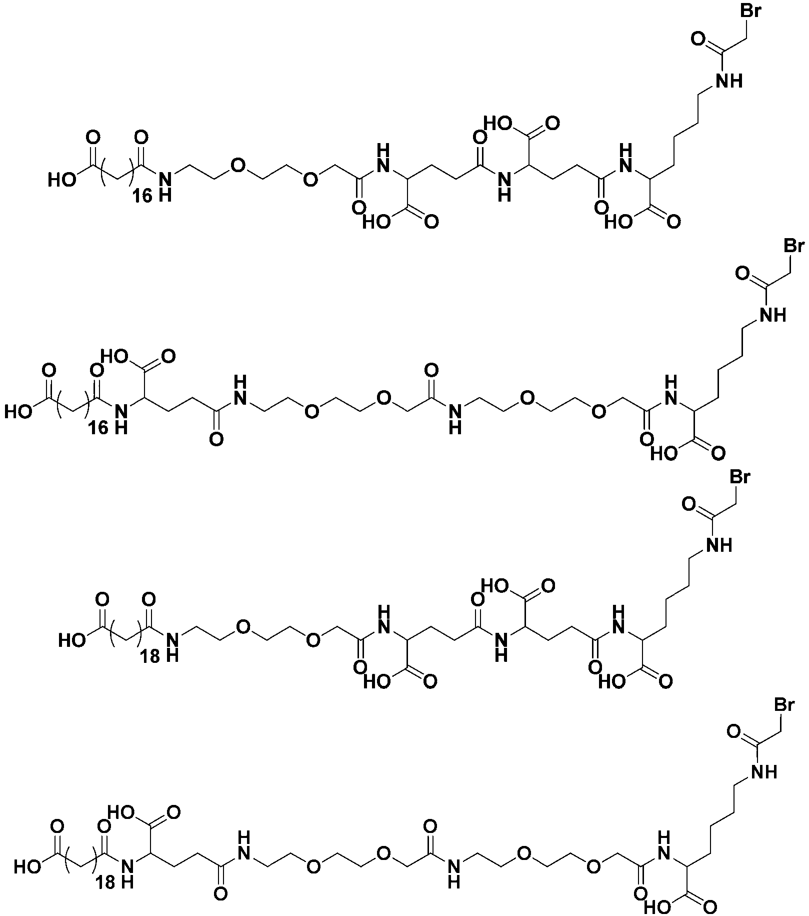

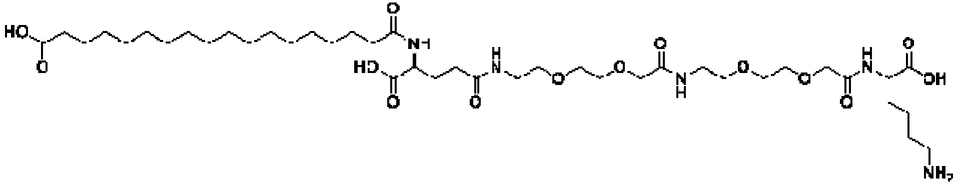

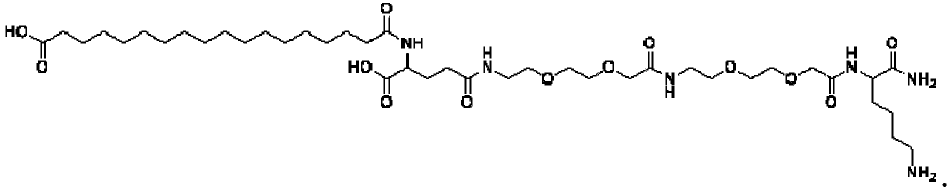

In a specific embodiment, the PAS is PAS₁₅-PAS₂₀₀; preferably PAS₁₅-PAS₁₀₀; for example, PAS₅₀.

In a preferred embodiment, the PAS₅₀ is shown in SEQ ID NO: 11.

In a preferred embodiment, a cysteine residue is inserted at position 3 of the N-terminus of the PAS₅₀.

In a preferred embodiment, the PAS can be replaced with a linker sequence (GSSSS)ₚ, wherein p is an integer selected from 1-3.

In a specific embodiment, the connector is a multivalent connector, thereby connecting one or more albumin-binding agents and one or more protein moieties.

In a preferred embodiment, the multivalent connector connects one albumin-binding agent and multiple protein moieties.

In a specific embodiment, the structure of the multivalent connector is shown in the following formula: 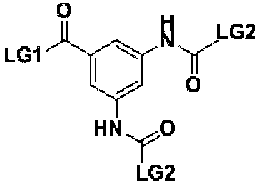 wherein
LG1 is a leaving group reactive to primary amino groups; and
LG2 is a leaving group reactive to sulfhydryl groups.

In a preferred embodiment, LG1 is a leaving group reactive to Lys.

In a preferred embodiment, LG2 is a leaving group reactive to Cys.

In a preferred embodiment, LG1 is connected to the albumin-binding agent, and LG2 is connected to the protein moiety.

In a preferred embodiment, LG1 is selected from active ester groups commonly used in peptide synthesis, including but not limited to: N-hydroxysuccinimide (NHS) ester group, sulfo-NHS ester group, pentafluorophenol (PFP) ester group, p-nitrophenol (PNP) ester group, hydroxybenzotriazole (HOBt) ester group, and (hydroxyimino) cyanoacetate ethyl ester (Oxyma) group.

In a preferred embodiment, LG1 is a carboxylic acid moiety or hydroxyl group activated by succinimide ester (OSu ester).

In a preferred embodiment, LG2 is selected from halogens, such as an iodine, bromine, or chlorine.

In a preferred embodiment, the multivalent connector is shown in the following formula:

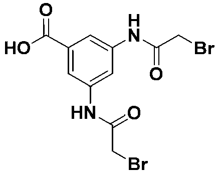

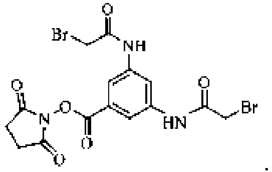

In a preferred embodiment, the albumin-binding agent and the multivalent connector form the following structure:

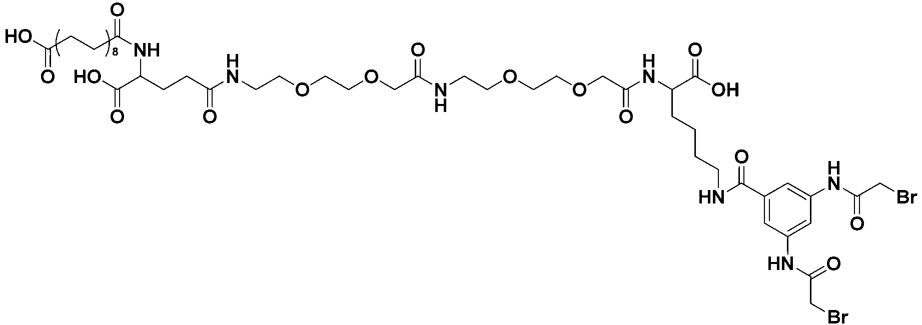

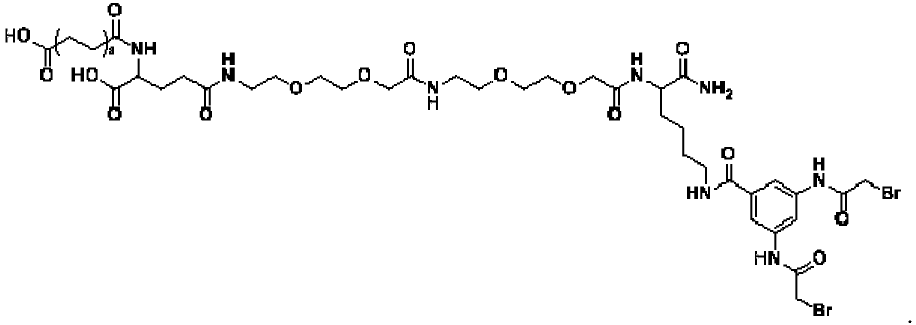

In a specific embodiment, the modified protein is selected from the compounds with the following numbers: 5.1, 5.2, 5.3, 5.4, 5.5, 5.6, 5.7, 5.8, 5.9, 5.10, 5.11, 5.12, 5.13, 5.14, 5.15, 7.2, 7.3, 7.4, 7.5, 12.1, 12.2, 12.3, 12.4, 12.5, 12.6.

In a preferred embodiment, the modified protein is selected from the compounds with the following numbers: 5.3, 5.4, 5.5, 5.15, 7.4, 7.5, 12.1, 12.2, 12.3, 12.4, 12.5, 12.6.

In the second aspect, the present invention provides a protein polymer comprising a multivalent connector and multiple protein monomers, wherein the protein monomers are optionally linked to PAS at their N-terminals, the multivalent connector is connected to the cysteine residues in the protein monomers or the cysteine residues in the PAS, and the cysteine residues are either naturally occurring or introduced through mutation.

In a preferred embodiment, the protein polymer comprises 2-3; preferably 2 protein monomers.

In a preferred embodiment, in the protein polymer, the multivalent connector is connected to the cysteine residue in the PAS.

In a specific embodiment, the PAS is PAS₁₅-PAS₂₀₀; preferably PAS₁₅-PAS₁₀₀; for example, PAS₅₀.

In a preferred embodiment, the PAS can be replaced with a linker sequence (GSSSS)ₚ, wherein p is an integer selected from 1-3.

In a preferred embodiment, the mutation is a substitution or insertion; preferably, a substitution.

In a preferred embodiment, the protein is a cell growth factor, growth hormone, interferon, enzyme, antibody, or an active fragment thereof.

In a preferred embodiment, the cell growth factor is epidermal fibroblast growth factor, platelet-derived fibroblast growth factor, fibroblast growth factor, insulin-like fibroblast growth factor, nerve fibroblast growth factor, interleukin-like fibroblast growth factor, erythropoietin, or colony-stimulating factor.

In a specific embodiment, the fibroblast growth factor is fibroblast growth factor 21.

In a preferred embodiment, the amino acid sequence of the fibroblast growth factor 21 is shown in SEQ ID NO: 1 or 2.

In a preferred embodiment, the amino acid sequence of the fibroblast growth factor 21 is shown in SEQ ID NO: 1 or 2, and cysteine residues are introduced therein through mutation.

In a preferred embodiment, the amino acid sequence of the fibroblast growth factor 21 is shown in SEQ ID NO: 1 or 2, and Q28, E30, D38, D46, K122 or H125 is mutated to a cysteine residue.

In a preferred embodiment, the amino acid sequence of the fibroblast growth factor 21 is shown in any one of SEQ ID NO: 4-9.

In a preferred embodiment, the amino acid sequence of the fibroblast growth factor 21 is shown in any one of SEQ ID NO: 5-9.

In a specific embodiment, the structure of the multivalent connector is shown in the following formula: 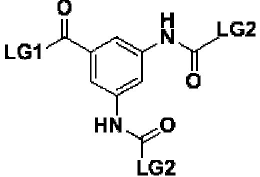 wherein
LG1 is a leaving group reactive to primary amino groups; and
LG2 is a leaving group reactive to sulfhydryl groups.

In a preferred embodiment, LG1 is a leaving group reactive to Lys.

In a preferred embodiment, LG2 is a leaving group reactive to Cys.

In a preferred embodiment, LG1 is connected to the albumin-binding agent, and LG2 is connected to the protein moiety.

In a preferred embodiment, LG1 is selected from active ester groups commonly used in peptide synthesis, including but not limited to: N-hydroxysuccinimide (NHS) ester group, sulfo-NHS ester group, pentafluorophenol (PFP) ester group, p-nitrophenol (PNP) ester group, hydroxybenzotriazole (HOBt) ester group, and (hydroxyimino) cyanoacetate ethyl ester (Oxyma) group.

In a preferred embodiment, LG1 is a carboxylic acid moiety or hydroxyl group activated by succinimide ester (OSu ester).

In a preferred embodiment, LG2 is selected from halogens, such as an iodine, bromine, or chlorine.

In a preferred embodiment, the multivalent connector is shown in the following formula:

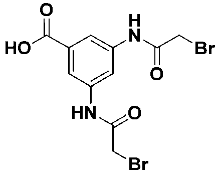

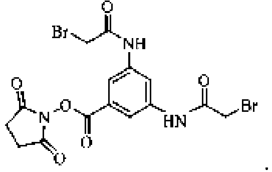

In a preferred embodiment, the protein polymer is selected from the compounds with the following numbers: 10.1, 10.2, 10.3, 10.4, 10.5.

In the third aspect, the present invention provides a pharmaceutical composition, comprising the modified protein described in the first aspect or the protein polymer described in the second aspect, and a pharmaceutically acceptable excipient.

In a preferred embodiment, the pharmaceutical composition is used for non-alcoholic steatohepatitis, type 2 diabetes, hyperlipidemia, and atherosclerosis; preferably non-alcoholic steatohepatitis.

In the fourth aspect, the present invention provides a use of the modified protein described in the first aspect or the protein polymer described in the second aspect in the preparation of a medicament.

In a preferred embodiment, the medicament is used for non-alcoholic steatohepatitis, type 2 diabetes, hyperlipidemia, and atherosclerosis; preferably non-alcoholic steatohepatitis.

In the fifth aspect, the present invention provides an albumin-binding agent, and the structure of the albumin-binding agent is shown in the following formula:

R₁-(X₁)ₘ-(X₂)ₙ-K(ε-R₂);

wherein K is a lysine;
R₁ is a substituted or unsubstituted C₁₋₂₀ acyl;
m is an integer from 0 to 5;
n is an integer from 0 to 5;
X₁ and X₂ are independently selected from the following group: alanine (Ala), D-alanine (D-Ala), β-alanine (β-Ala), 4-aminobutyric acid (GABA), 2-aminoisobutyric acid (Aib), 2-aminobutyric acid (Abu), arginine (Arg), aspartic acid (Asp), asparagine (Asn), cysteine (Cys), glutamic acid (Glu), D-glutamic acid (D-Glu), γ-glutamic acid (γ-Glu), glutamine (Gln), glycine (Gly), histidine (His), isoleucine (Ile), leucine (Leu), lysine (Lys), proline (Pro), phenylalanine (Phe), serine (Ser), tyrosine (Tyr), threonine (Thr), tryptophan (Trp), valine (Val), methionine (Met), tranexamic acid (Trx), AEEA, PEG;
R₂ is selected from a halogen-substituted C₁₋₆ acyl or H.

In a preferred embodiment, R₁ is selected from: a heptanoyl, methylheptanoyl, octanoyl, methyloctanoyl, nonanoyl, methylnonanoyl, decanoyl, methyldecanoyl, lauroyl, myristoyl, palmitoyl, stearoyl, 17-carboxyheptadecanoyl, 15-carboxypentadecanoyl, 13-carboxytridecanoyl, 11-carboxyundecanoyl.

In a preferred embodiment, R₁ is selected from: a lauroyl, myristoyl, palmitoyl, stearoyl, 17-carboxyheptadecanoyl, 19-carboxynonadecanoyl.

In a preferred embodiment, m is an integer from 1 to 3.

In a preferred embodiment, n is an integer from 1 to 3.

In a preferred embodiment, X₁ and X₂ are independently selected from the group consisting of 4-aminobutyric acid (GABA), 2-aminoisobutyric acid (Aib), D-alanine (D-Ala), β-alanine (β-Ala), aspartic acid (Asp), cysteine (Cys), glutamic acid (Glu), γ-glutamic acid (γ-Glu), glycine (Gly), serine (Ser), and tyrosine (Tyr).

In a preferred embodiment, X₁ and X₂ are independently selected from the group consisting of glutamic acid (Glu), γ-glutamic acid (γ-Glu), and tyrosine (Tyr).

In a preferred embodiment, R₂ is selected from a halogen-substituted C₁₋₃ acyl or H.

In a preferred embodiment, R₂ is selected from an iodoacetyl, bromoacetyl, or H; more preferably bromoacetyl or H.

In a specific embodiment, the albumin-binding agent is selected from the group consisting of:

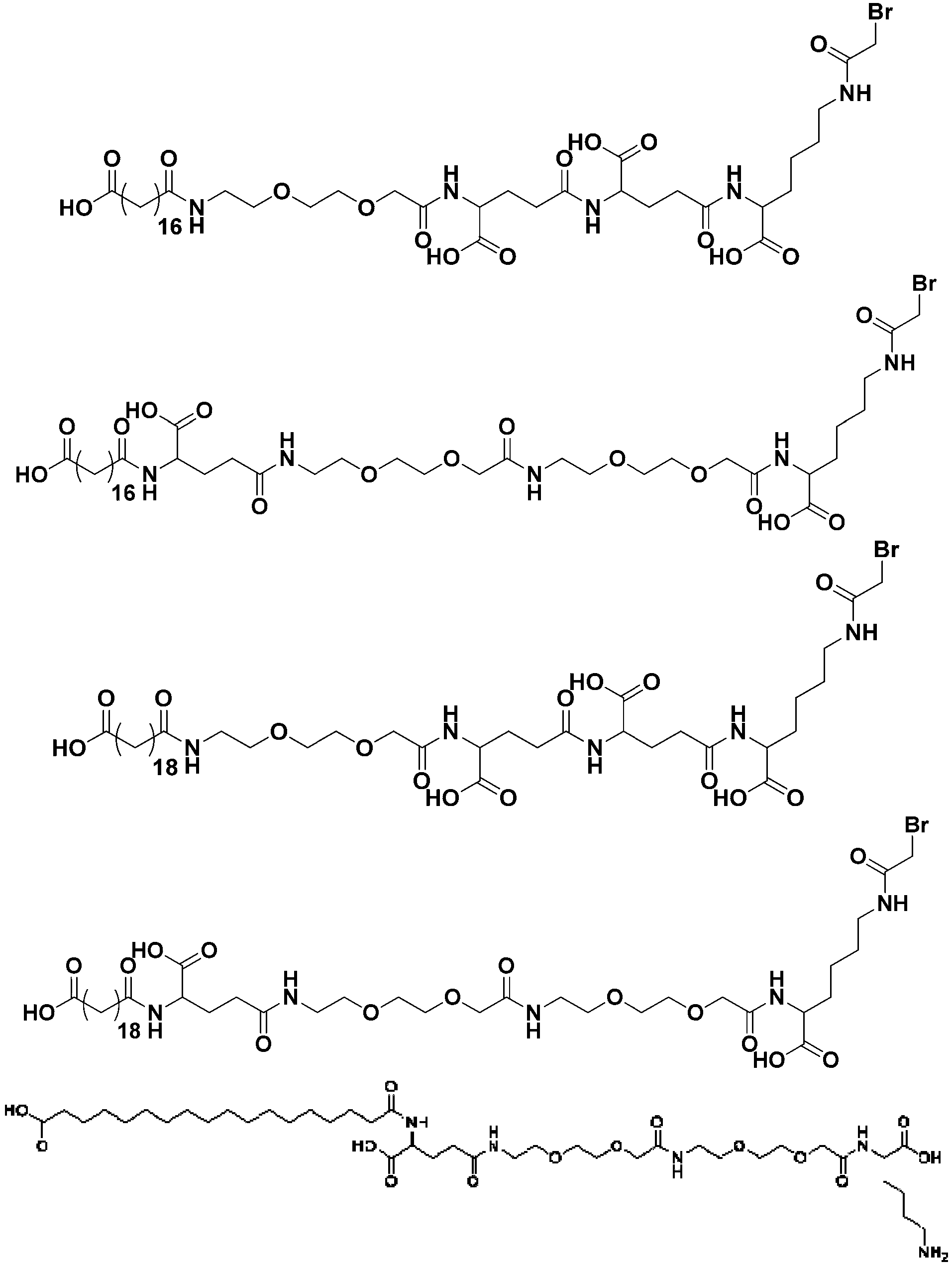

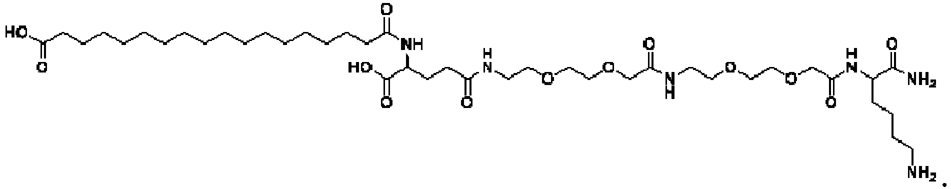

In the sixth aspect, the present invention provides a multivalent connector, and the structure of the multivalent connector is shown in the following formula: 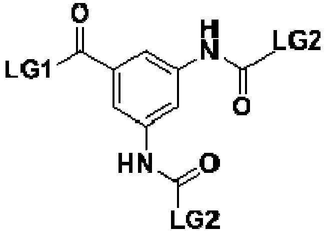 wherein
LG1 is a leaving group reactive to primary amino groups; and
LG2 is a leaving group reactive to sulfhydryl groups.

In a preferred embodiment, LG1 is a leaving group reactive to Lys.

In a preferred embodiment, LG2 is a leaving group reactive to Cys.

In a preferred embodiment, LG1 is connected to the albumin-binding agent, and LG2 is connected to the protein moiety.

In a preferred embodiment, LG1 is selected from active ester groups commonly used in peptide synthesis, including but not limited to: N-hydroxysuccinimide (NHS) ester group, sulfo-NHS ester group, pentafluorophenol (PFP) ester group, p-nitrophenol (PNP) ester group, hydroxybenzotriazole (HOBt) ester group, and (hydroxyimino) cyanoacetate ethyl ester (Oxyma) group.

In a preferred embodiment, LG1 is a carboxylic acid moiety or hydroxyl group activated by succinimide ester (OSu ester).

In a preferred embodiment, LG2 is selected from halogens, such as an iodine, bromine, or chlorine.

In a preferred embodiment, the multivalent connector is shown in the following formula:

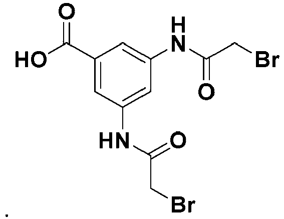

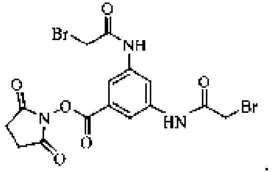

In the seventh aspect, the present invention provides a polypeptide, comprising the amino acid sequence shown in SEQ ID NO: 11.

In the eighth aspect, the present invention provides a polypeptide, the amino acid sequence of which is shown in SEQ ID NO: 11, but with a cysteine residue inserted at position 3 of the N-terminus.

In the ninth aspect, the present invention provides a polypeptide, comprising an amino acid sequence shown in any one of SEQ ID NO: 4-9; preferably an amino acid sequence shown in any one of SEQ ID NO: 5-9.

In the tenth aspect, the present invention provides an isolated nucleic acid molecule, encoding the polypeptide described in any one of the seventh to ninth aspects.

In the eleventh aspect, the present invention provides an expression vector, comprising the isolated nucleic acid molecule described in the tenth aspect.

In the twelfth aspect, the present invention provides a host cell, comprising the expression vector described in the eleventh aspect, or having the nucleic acid molecule described in the tenth aspect integrated into the genome of the host cell.

In the thirteenth aspect, the present invention provides a use of the polypeptide described in the tenth to twelfth aspects in the preparation of the modified protein described in the first aspect, the protein polymer described in the second aspect, or the pharmaceutical composition described in the third aspect.

In the fourteenth aspect, the present invention provides a therapeutic method, comprising a step of administering a therapeutically effective amount of the modified protein described in the first aspect, the protein polymer described in the second aspect, or the pharmaceutical composition described in the third aspect to a subject in need thereof.

In a preferred embodiment, the therapeutic method is used for non-alcoholic steatohepatitis, type 2 diabetes, hyperlipidemia, and atherosclerosis; preferably non-alcoholic steatohepatitis.

It should be understood that within the scope of the present invention, the above-mentioned technical features of the present invention and the technical features specifically described below (such as in the examples) can be combined with each other to form new or preferred technical solutions, which will not be repeated on by one herein due to the limited contents.

### Description of drawings

Figure 1 shows the pharmacokinetic curve in mice;
Figure 2 shows the pharmacokinetic curve in minipigs.

### Modes for carrying out the invention

After extensive and in-depth research, the inventors unexpectedly discovered analogs of the metabolic regulatory protein FGF21, thereby obtaining FGF21 analogs with potential for treating NASH. Such FGF21 analogs not only have excellent binding affinity with their receptors, but also have significantly improved *in vivo* half-life, based on which the present invention was completed.

Unless otherwise specified, the terms used herein have the meanings commonly understood by a skilled person. When trade names appear herein, they are intended to refer to the corresponding products or active ingredients therein. For the sake of clarity, some terms used herein are defined as follows:

The term "isolated nucleic acid molecule" used herein has the meaning commonly understood by a skilled person, which refers to: 1) being separated from at least about 50% of proteins, lipids, carbohydrates, or other materials that naturally coexist with it when total nucleic acids are isolated from the source cell; 2) not being connected to all or part of polynucleotides that are not naturally connected to the "isolated nucleic acid molecule"; 3) being effectively connected to polynucleotides that are not naturally connected to it; or 4) not naturally existing as part of a larger polynucleotide sequence. Preferably, the isolated nucleic acid molecule is substantially free of any other contaminating nucleic acid molecules or other pollutants present in its natural environment that may interfere with its use in polypeptide production or its therapeutic, diagnostic, preventive, or research uses.

The term "vector" used herein has the meaning commonly understood by a skilled person, referring to any molecule (such as nucleic acid, plasmid, or virus) used to deliver coding information to a host cell.

The term "expression vector" used herein has the meaning commonly understood by a skilled person, referring to a vector that is suitable for the transformation of host cells and comprises nucleic acid sequences that direct and/or control the expression of inserted heterologous nucleic acid sequences. The expression includes, but is not limited to, processes, such as transcription, translation, and RNA splicing (if introns are present).

The term "effective linked" as used herein has the meaning conventionally understood by a skilled person, which refers to the arrangement of flanking sequences, wherein the flanking sequences are configured or assembled to perform their conventional functions. Therefore, a flanking sequence that is effectively linked to a coding sequence may be able to achieve the replication, transcription, and/or translation of the coding sequence. For example, a coding sequence is effectively linked to a promoter when the promoter can direct the transcription of the coding sequence. The flanking sequences are not necessarily to be adjacent to the coding sequence as long as they function correctly. Thus, for example, an intervening untranslated but transcribed sequence can exist between a promoter sequence and a coding sequence, and the promoter sequence can still be considered "effectively linked" to the coding sequence.

The term "host cell" as used herein has the meaning conventionally understood by a skilled person, which refers to a cell that is transformed by a nucleic acid sequence (such as the nucleic acids provided herein) or capable of being transformed by said nucleic acid sequence and then able to express a selected target gene. The term includes the progeny of the parental cell, regardless of whether the progeny is identical to the original parent in terms of morphology or genetic composition, as long as the selected gene is present.

In the context of the present invention, the term "FGF21" as used herein refers to human fibroblast growth factor 21 and mutants of said FGF21. "FGF21" and "human fibroblast growth factor 21" as used herein are used interchangeably. The mutants shall be understood as compounds obtained by the following steps: substituting one or more amino acid residues in the FGF21 sequence with another natural or non-natural amino acid; and/or adding one or more natural or non-natural amino acids to the FGF21 sequence; and/or deleting one or more amino acid residues from the FGF21 sequence, where any of these steps may optionally be followed by further derivation of one or more amino acid residues. Specifically, a substitution is conservative when one amino acid residue is replaced by another from the same group (i.e., by another amino acid residue with similar properties). Amino acids can be appropriately divided into the following groups based on their properties: basic amino acids (e.g., arginine, lysine, histidine), acidic amino acids (e.g., glutamic acid and aspartic acid), polar amino acids (e.g., glutamine, cysteine, and asparagine), hydrophobic amino acids (e.g., leucine, isoleucine, proline, methionine, and valine), aromatic amino acids (e.g., phenylalanine, tryptophan, tyrosine), and small amino acids (e.g., glycine, alanine, serine, and threonine). Typically, a FGF21 mutant will have at least 80% identity with human FGF21.

"Basically" means "almost all" or "complete", such as meeting one or more of the following: more than 50%, 51% or more, 75% or more, 80% or more, 90% or more, and 95% or more of the cases.

As used herein, "sequence identity" is determined by: aligning and comparing the sequence of a reference DNA with another DNA sequence to maximize the overlap between the two sequences while minimizing sequence gaps, ignoring any overhanging sequences between the two sequences. For any sequence identity described herein, it is preferably at least 80%, more preferably 85%, even more preferably 90%, still more preferably 95% sequence identity, and most preferably 96%, 97%, 98%, and 99% sequence identity.

The abbreviations for amino acid residues in proteins are as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic acid is Asp or D; Glutamic acid is Glu or E; Cysteine is Cys or C; Tryptophan is Trp or W; Arginine is Arg or R; Glycine is Gly or G.

The term "optional" or "optionally" means that the subsequently described event or situation may or may not occur, and the description includes both the occurrence and non-occurrence of the said event or situation. For example, ethyl being "optionally" substituted by a halogen means that ethyl can be unsubstituted (CH₂CH₃), monosubstituted (such as CH₂CH₂F), polysubstituted (such as CHFCH₂F, CH₂CHF₂, etc.), or fully substituted (CF₂CF₃). A skilled person will understand that for any group containing one or more substituents, no substitutions or substitution patterns that are spatially impossible and/or cannot be synthesized will be introduced.

As used herein, Cₘ₋ₙ refers to a moiety with m-n carbon atoms in that part. For example, "C₀₋₆ alkylene" means that the alkylene has 0-6 carbon atoms, and when the alkylene has 0 carbon atoms, the group is a bond.

The numerical ranges as used herein refer to each integer within the given range. For example, "C₁₋₆" means that the group can have 1 carbon atom, 2 carbon atoms, 3 carbon atoms, 4 carbon atoms, 5 carbon atoms, or 6 carbon atoms.

When any variable (such as R) appears more than once in the composition or structure of a compound, its definition in each case is independent. Therefore, for example, if a group is substituted with 2 Rs, each R has independent options.

The term "substituted" refers to the replacement of any one or more hydrogen atoms on a specific atom by substituents, provided that the valence state of the specific atom is normal and the substituted compound is stable. When the substituent is oxo (i.e., =O), it means that two hydrogen atoms are replaced, and oxo substitution does not occur on aromatic groups.

The term "conjugate" as a noun refers to a modified protein, i.e., a protein with a moiety bonded to it to modify the properties of the protein; and as a verb, the term refers to the process of bonding a moiety to a protein to modify the properties of the protein.

The term "FGF21 fusion protein" as used herein refers to the fusion of one or more amino acid residues (such as heterologous proteins or peptides) to the N-terminus or C-terminus of any FGF21 polypeptide mutant described herein. Heterologous peptides and polypeptides include, but are not limited to, epitopes that can be used to detect and/or isolate FGF21 polypeptide mutants; transmembrane receptor proteins or parts thereof, such as extracellular domains or transmembrane and intracellular domains; ligands that bind to transmembrane receptor proteins or parts thereof; enzymes or catalytically active parts thereof; polypeptides or peptides that promote oligomerization, such as leucine zipper domains; polypeptides or peptides that improve stability, such as immunoglobulin constant regions (e.g., domains); half-life extension sequences, comprising combinations of two or more (e.g., 2, 5, 10, 15, 20, 25, etc.) naturally occurring or non-naturally occurring, charged and/or uncharged amino acids (e.g., serine, glycine, glutamic acid, or aspartic acid), designed to form fusion partners of predominantly hydrophilic or predominantly hydrophobic mutants; functional or non-functional antibodies, or heavy or light chains thereof; and polypeptides with activities different from those of the FGF21 polypeptide mutants of the present invention, such as therapeutic activities. FGF21 fusion proteins can be prepared by fusing a heterologous sequence to the N-terminus or C-terminus of an FGF21 polypeptide mutant. The heterologous sequences described herein can be amino acid sequences or polymers comprising non-amino acids. The heterologous sequence can be fused directly or via a linker or adapter molecule to the FGF21 polypeptide mutant. The linker or adapter molecule can be one or more amino acid residues (or amino acid polymers), such as 1, 2, 3, 4, 5, 6, 7, 8, or 9 residues (or amino acid polymers), preferably 10-50 amino acid residues (or amino acid polymers), such as 10, 11, 12, 13, 14, 15, 16, 17, 18, 19, 20, 25, 30, 35, 40, 45, or 50 residues (or amino acid polymers), more preferably 15-35 amino acid residues (or amino acid polymers). The linker or adapter molecule can also be designed to have cleavage sites for DNA restriction endonucleases or proteases for separating the fusion parts.

The terms "analog" or "derivative" mentioned herein in relation to FGF21, namely FGF21 analogs or FGF21 derivatives, refer to polypeptides derived or derivable from natural FGF21, particularly from or derivable from SEQ ID NO: 1. That is, such modifications, amendments, or changes through the modification of amino acid sequences may include one or more amino acid substitutions, deletions, and/or additions. For example, amino acids can be added and/or deleted at the C-terminal, N-terminal, or internally in the amino acid sequence. It is preferred to add and/or delete amino acids at the C-terminal and/or N-terminal, more preferably at the N-terminal. Amino acid sequences with amino acid deletions at C-terminal or N-terminal may also be referred to as truncated sequences, which are known in the art. Similarly, amino acids added internally in the sequence may be referred to as insertions.

A "side chain" is a chemical unit that does not possess special properties, especially biological activity. In one or more embodiments of the present invention, the side chains include albumin-binding agent side chains and multivalent linker side chains.

The term "albumin-binding agent" itself is not restrictive but descriptive, as it reflects the overall goal or purpose of linking to FGF21, that is, the resulting conjugate (or analog) can bind to human serum albumin, and the purpose of such binding is to provide or at least contribute to a sustained effect for the analogs of the present invention. If necessary, this term can also be replaced with other general chemical terms, such as compound.

"Multivalent connector": in the context of the present invention, a connector is a chemical moiety or residue used to covalently link the proteins in question. When the connector reacts with the protein, a connector group is formed. Therefore, the term "-connector-" is intended to denote the chemical unit of the conjugate, which is covalently linked to the amino acid residues of each polypeptide of the protein conjugate. However, by using multivalent connectors (including bivalent connectors and trivalent connectors) according to the present invention, FGF21 dimers are obtained.

"PAS" is used to describe a repeating amino acid sequence composed of proline (P), alanine (A), and serine (S). It is an amino acid sequence consisting of 0 to approximately 400 amino acid residues, such as about 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400. The number of amino acids in a PAS is indicated in the form of a subscript; for example, PAS₁₀ refers to 10 PAS units composed of proline (P), alanine (A), and serine (S) residues. In some schemes, the proline residues of the PAS account for 10% - 40% of the total amino acid residues of the polypeptide PAS. In a specific embodiment, the PAS is PAS₁₅-PAS₂₀₀; preferably PAS₁₅-PAS₁₀₀; for example, PAS₅₀.

In the context of the present invention, the term "pharmaceutically acceptable salt" refers to a salt that is harmless to patients. Such salts include pharmaceutically acceptable acid addition salts, pharmaceutically acceptable metal salts, ammonium salts, and alkylammonium salts. Acid addition salts include salts of inorganic acids and organic acids. Representative examples of suitable inorganic acids include hydrochloric acid, hydrobromic acid, hydroiodic acid, phosphoric acid, sulfuric acid, nitric acid, and the like. Representative examples of suitable organic acids include formic acid, acetic acid, trichloroacetic acid, trifluoroacetic acid, propionic acid, benzoic acid, cinnamic acid, citric acid, fumaric acid, glycolic acid, lactic acid, maleic acid, malic acid, malonic acid, mandelic acid, oxalic acid, picric acid, pyruvic acid, salicylic acid, succinic acid, methanesulfonic acid, ethanesulfonic acid, tartaric acid, ascorbic acid, pamoic acid, dimethylenesalicylic acid, ethanedisulfonic acid, gluconic acid, citric acid, aspartic acid, stearic acid, palmitic acid, EDTA, glycolic acid, p-aminobenzoic acid, glutamic acid, benzenesulfonic acid, p-toluenesulfonic acid, and the like. In addition, metal salts include lithium salts, sodium salts, potassium salts, magnesium salts, and the like. Ammonium salts and alkylammonium salts include ammonium salts, methylammonium salts, dimethylammonium salts, trimethylammonium salts, ethylammonium salts, hydroxyethylammonium salts, diethylammonium salts, butylammonium salts, tetramethylammonium salts, and the like.

The present application also includes isotopically labeled compounds of the application that are identical to those described herein, but with one or more atoms being replaced by atoms having an atomic weight or mass number different from the atomic weight or mass number usually found in nature. Examples of isotopes that can be incorporated into the compounds of the application include isotopes of hydrogen, carbon, nitrogen, oxygen, phosphorus, sulfur, fluorine, iodine, and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively.

Certain isotopically labeled compounds of the present application (for example, those labeled with ³H and ¹⁴C) can be used in compound and/or substrate tissue distribution analysis. Tritiated (i.e., ³H) and carbon-14 (i.e., ¹⁴C) isotopes are particularly preferred because they are easy to be prepared and detectable. In addition, substitution with heavier isotopes (such as deuterium (i.e., ²H)) can provide certain therapeutic advantages resulting from higher metabolic stability (e.g., increased *in vivo* half-life or reduced dosage requirements), and thus may be preferred in some cases. Positron-emitting isotopes, such as ¹⁵O, ¹³N, ¹¹C, and ¹⁸F, can be used in positron emission tomography (PET) studies to determine substrate occupancy. Isotopically labeled compounds of the present application can generally be prepared by substituting non-isotopically labeled reagents with isotopically labeled reagents through procedures similar to those disclosed in the schemes and/or examples below.

Therapeutic uses of FGF21 analogs: FGF21 analogs can be used to treat, diagnose, improve, or prevent various diseases, disorders, or conditions, including but not limited to metabolic disorders, including diabetes; dyslipidemia; hypertension; hepatic steatosis, such as non-alcoholic steatohepatitis (NASH); cardiovascular diseases, such as atherosclerosis.

In applications, conditions or diseases such as diabetes or obesity can be treated by administering the FGF21 polypeptide mutants described herein to patients in need thereof in a therapeutically effective dose. Administration can be performed according to the methods described herein, such as by intravenous injection, intraperitoneal injection, intramuscular injection, or oral administration in the form of tablets or liquid preparations. In most cases, the required dose can be determined by a clinical staff as described herein and may represent a therapeutically effective dose of the FGF21 mutant polypeptide. It will be apparent to a skilled person that the therapeutically effective dose of the FGF21 mutant polypeptide will depend, inter alia, on the administration schedule, the unit dose of the substance administered (whether the nucleic acid molecule or polypeptide is administered in combination with other therapeutic agents), the immune status, and the health status of the recipient. As used herein, the term "therapeutically effective dose" refers to the amount of FGF21 mutant polypeptide that researchers or other clinicians seek to elicit a biological or pharmacological response in a tissue system, animal, or human, including alleviating the symptoms of the disease or condition being treated.

In a specific embodiment, the FGF21 mutant of the present invention comprises an amino acid sequence as shown in any one of SEQ ID NO: 4-9, preferably as shown in any one of SEQ ID NO: 5-9.

### Modified protein of the present invention

In the present invention, by conjugating protein drugs with specific chemical molecules or domains (such as ABD) that can bind to proteins with long half-lives in the body, the *in vivo* half-life of the drugs can be extended, while having little impact on the biodistribution of the drugs. The proteins with long half-lives in the body are proteins in plasma, preferably human serum albumin.

When the protein with a long half-life in the body mentioned is human serum albumin, the specific chemical molecule capable of binding to the protein with a long half-life in the body is an albumin-binding agent. The albumin-binding agent is a substance that can specifically or non-specifically bind to human serum albumin, such as fatty acids.

In a specific embodiment, the structure of the albumin-binding agent of the present invention is shown in the following formula:

R₁-(X₁)ₘ-(X₂)ₙ-K(ε-R₂);

Wherein K, R₁, m, n, X₁, X₂ and R₂ are described as above.

Based on the albumin-binding agent as said above, the present invention provides such a modified protein, in which the albumin-binding agent is linked to a cysteine residue in the protein portion. Based on the teachings of the present invention, a skilled person will know that the cysteine residue can be naturally occurring or introduced through mutation. The method of mutation can be a substitution or insertion, but the substitution is preferred.

In the modified protein of the present invention, the protein portion may also have a PAS linked to its N-terminus. The meaning of "PAS" here is as explained above. In a specific embodiment, the PAS contained at the N-terminus of the modified protein of the present invention is PAS₅₀; preferably as shown in SEQ ID NO: 11. Based on the teachings of the present invention, a skilled person will understand that the albumin-binding agent can also be linked to the protein portion of the modified protein through the PAS. Therefore, to connect with the albumin-binding agent, a cysteine residue can be introduced into the PAS by means of mutation (preferably insertion). In a preferred embodiment, a cysteine residue is inserted at position 3 of the N-terminus of the PAS₅₀.

The inventors have further discovered that in the modified protein of the present invention, the modifying moiety can also be linked to a cysteine residue in the protein moiety or a cysteine residue in the PAS via a connector, where the cysteine residue is either naturally occurring or introduced through mutation. The connector can be a multivalent connector, which can thus connect one or more albumin-binding agents and one or more protein moieties. Preferably, in the modified protein of the present invention, one albumin-binding agent and multiple protein moieties are connected via a multivalent connector. The specific structure of the multivalent connector is as shown above.

Based on the teachings of the present invention, a skilled person will know how to obtain the modified proteins of the present invention. For example, the albumin-binding agent and connector of the present invention can be obtained through conventional synthesis techniques, the protein moieties and PAS-linked protein moieties can be obtained through conventional biotechnology, and then the albumin-binding agent can be linked to the protein moieties either directly or via the connector. For example, a skilled person can obtain nucleic acid molecules encoding the protein moieties or PAS in the modified proteins of the present invention by conventional technical means, and subsequently obtain the protein moieties or PAS-connected protein moieties using host cells through conventional biotechnological means. The present invention also includes such nucleic acid molecules, or expression vectors comprising said nucleic acid molecules, as well as host cells.

After the modified protein of the present invention is prepared, the modified protein can be purified by techniques known in the art. Such techniques include, but are not limited to, reversed-phase high-performance preparative liquid chromatography, ion exchange chromatography, gel electrophoresis, affinity chromatography, size exclusion chromatography, etc. Actual conditions for protein purification will depend, in part, on factors such as net charge, hydrophobicity, hydrophilicity, etc., and will be obvious to a skilled person. High-performance preparative liquid chromatography can be used to separate the modified protein. The purity of the modified protein can be determined by any known analytical methods, including gel electrophoresis, high-performance liquid chromatography, etc., and the molecular weight can be verified by mass spectrometry.

In a further embodiment, the present invention provides an FGF21 fusion protein with an N-terminal PAS₅₀ modification. The N-terminal PAS₅₀-modified FGF21 fusion protein has been successfully prepared, and its cellular activity has been determined using a reporter gene assay. As is well known to a skilled person, compounds linked by disulfide bonds, including but not limited to Cys obtained by reaction and adduction with free Cys of the FGF21 fusion protein, reduced glutathione, cysteamine, and other such compounds, are also included within the scope of the FGF21 fusion protein.

Based on the FGF21 fusion protein modified with PAS₅₀ at the N-terminus, the inventors have obtained FGF21 conjugates modified with albumin-binding agent side chains.

The albumin-binding agent of the present invention can be linked to the protein moiety, such as the PAS₅₀-modified FGF21 protein, through a connector, for example, a multivalent connector. In a specific embodiment, the albumin-binding agent is linked to the FGF21 fusion protein with a PAS₅₀ modification at the N-terminus via a multivalent connector.

In a specific embodiment, the modified protein of the present invention is selected from the compounds with the following numbers: 5.1, 5.2, 5.3, 5.4, 5.5, 5.6, 5.7, 5.8, 5.9, 5.10, 5.11, 5.12, 5.13, 5.14, 5.15, 7.2, 7.3, 7.4, 7.5, 12.1, 12.2, 12.3, 12.4, 12.5, 12.6; preferably the compounds with the following numbers: 5.3, 5.4, 5.5, 5.15, 7.4, 7.5, 12.1, 12.2, 12.3, 12.4, 12.5, 12.6, and their biological activity is identified by the reporter gene method.

Using the multivalent connector of the present invention, the inventors have also obtained a protein polymer, comprising multiple protein monomers connected by the multivalent connector. The protein monomer is optionally linked with PAS at the N-terminus, and the multivalent connector is connected to the cysteine residue in the protein moiety or the cysteine residue in the PAS, and the cysteine residue is either naturally occurring or introduced through mutation.

The number of protein monomers in the protein polymer can be determined by a skilled person according to actual circumstances. For example, the protein polymer of the present invention may comprise 2-3 protein monomers; preferably 2 protein monomers.

A skilled person will know that the PAS in the protein polymer can also be replaced with a linker sequence (GSSSS)ₚ, wherein p is an integer selected from 1-3.

In a specific embodiment, the structure of the multivalent connector is as shown in the following formula:

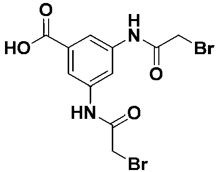

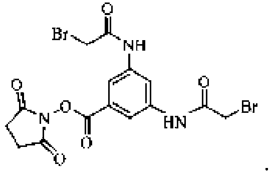

In a specific embodiment, the protein polymer of the present invention is selected from the compounds with the following numbers: 10.1, 10.2, 10.3, 10.4, 10.5.

### Advantages of the invention:

1. The present invention provides a novel modified protein or protein polymer;
2. The modified protein or protein polymer of the present invention retains original activities of the protein, and its *in vivo* half-life is also significantly improved;
3. The preparation process of the modified protein or protein polymer of the present invention is simple, and it can be conveniently obtained through technical means such as biotechnology and chemical synthesis technology; and
4. The modified protein or protein polymer of the present invention has laid a new material foundation for the development of therapeutic medicaments suitable for non-alcoholic steatohepatitis, type 2 diabetes, hyperlipidemia, atherosclerosis and other diseases.

Following specific examples will further illustrate the present invention. It should be understood that these examples are only used to illustrate the present invention and not to limit the scope of the present invention. For the experimental methods without specified conditions in the following examples, conventional conditions are usually followed, such as those described in (Sambrook and Russell et al., Molecular Cloning-A Laboratory Manual (3rd Edition) (2001) CSHL Press), or the conditions recommended by manufacturers. Unless otherwise specified, percentages and parts are calculated by weight. Experimental materials and reagents used in the following examples can be obtained from commercial channels unless otherwise specified.

### Example

Abbreviations used in the following examples have meanings commonly understood by a skilled person. For the sake of clarity, these abbreviations are explained as follows:
Tris-HCl: Tris(hydroxymethyl)aminomethane hydrochloride
SDS: Sodium dodecyl sulfate
DMF: N,N-Dimethylformamide
DTT: Dithiothreitol
EDTA: Ethylenediaminetetraacetic acid
TFA: Trifluoroacetic acid
HSA: Human serum albumin
PBS: Potassium dihydrogen phosphate (KH₂PO₄) 0.24g/L, disodium hydrogen phosphate (Na₂HPO₄) 1.44g/L, sodium chloride (NaCl) 8g/L, potassium chloride (KCl) 0.2g/L, pH 7.4
TCEP: Tris(2-carboxyethyl)phosphine
Tris: Tris(hydroxymethyl)aminomethane or 2-amino-2-hydroxymethylpropane-1,3-diol
LCMS: Liquid chromatography-mass spectrometry
AEEA: 2-(2-(2-aminoethoxy)ethoxy)acetic acid
RP-HPLC: Reversed-phase high-performance liquid chromatography
DCM: Dichloromethane
Fmoc: 9-Fluorenylmethoxycarbonyl
DIEA: N,N-Diisopropylethylamine
HOBt: 1-Hydroxybenzotriazole
DIC: N,N-Diisopropylcarbodiimide
PIP: Piperidine

### General Detection and Characterization Methods

### RP-HPLC

| | |
|---|---|
| Chromatographic system | Waters Alliance HPLC |
| Chromatographic column | YMC Triart Bio C4 5µm |
| Column temperature | 50°C |
| Flow rate | 0.8 ml/min |
| Mobile phase A | 0.1%TFA(v/v) aqueous solution |
| Mobile phase B | 0.1%TFA(v/v) acetonitrile solution |
| Gradient | 34%-35% 10 min; 35%-40% 5 min; 40%-60% 5 min |

### LC-MS method

| | |
|---|---|
| Mass spectrometry system | Waters Xevo Q-Tof |
| Chromatographic system | Waters Acquity UPLC |
| Chromatographic column | YMC Triart Bio C4 1.9µm |
| Column temperature | 40°C |
| Flow rate | 0.4 ml/min |
| Mobile phase A | 0.1% formic acid (v/v) aqueous solution |
| Mobile phase B | 0.1% formic acid (v/v) acetonitrile solution |
| Gradient | 5%-95% 10min |

### Example 1: Preparation of FGF21 and mutants thereof

The sequence of mature human FGF21 is publicly available and can be queried in the UniProt database under the accession number Q9NSA1, as shown in SEQ ID NO: 1. Literatures, such as PLoS One. 2012; 7(11): e49345; Endocrinology. 2017; 158(5): 1314-1327 reported that the L98R, P171G, and A180E mutations effectively enhance the anti-aggregation and anti-degradation capabilities of FGF21. The preparation process of this mutant (SEQ ID NO: 2), i.e., FGF21RGE, is described as follows.

(1) The expression of FGF21RGE was carried out in the form of a tagged fusion protein. An expression cassette containing an N-terminal 6×histidine (His₆) tag and a small ubiquitin-like modifier (SUMO) tag for FGF21RGE expression was constructed and inserted into the pET30 plasmid. It was then transformed into competent *E. coli* BL21(DE3) bacteria, and an engineered strain for expressing the His₆-SUMO-FGF21RGE (SEQ NO: 3) fusion protein was obtained through screening. After high-density fermentation of the engineered bacteria, the expressed His₆-SUMO-FGF21RGE fusion protein was expressed at a high level in a soluble form in the cytoplasm.

(2) First, the soluble fusion protein His₆-SUMO-FGF21RGE (SEQ NO: 3) was purified, then the soluble target FGF21RGE protein was obtained through enzymatic cleavage, and further purified to obtain the soluble target FGF21RGE protein with high purity. Firstly, high-pressure homogenization was used to break the cells and release the intracellularly expressed soluble fusion protein; then the pH of the turbid homogenate was adjusted to 3.0, the homogenate was centrifuged, and then the pH was adjusted to 8.0; the fusion protein was enriched using metal ion chelate chromatography (IMAC) with HisTrap Ni excel (5 ml, purchased from Cytiva), and the fusion protein comprising the His₆-SUMO tag was eluted with 250 mM imidazole; then ULP1 enzyme (which can specifically cleave the SUMO tag) was added to the elute for enzymatic cleavage. The enzymatic cleavage system contains 0.2 mM TCEP, and the enzymatically cleaved solution was purified using anion exchange chromatography (AEX) such as HiTrap Q HP (5 ml, purchased from Cytiva). The pH for chromatography was maintained at 8.0, and elution was performed with a linear salt gradient of 0 - 500 mM NaCl. After SDS-PAGE analysis and RP-HPLC analysis, the soluble target FGF21RGE protein with high purity was obtained and pooled. Finally, the FGF21RGE protein was ultrafiltered into PBS against a 10 kDa MWCO membrane.

After LC-MS analysis, the actual molecular weight of FGF21RGE prepared according to the above method was 19453.0 Da, which is consistent with the theoretical molecular weight (19453.8 Da).

In the subsequent examples, various FGF21 proteins, including FGF21RGE and FGF21 mutants, etc., were expressed in a bacterial expression system such as *E. coli* BL21(DE3). Unless otherwise specified, the expression and purification will be carried out in accordance with the method described in this example.

### Example 2: Synthesis of modifying side chains

### 2.1 Synthesis of C18 Dicarboxylic Acid-AEEA-gGlu-gGlu-Lys(bromoacetyl)-COOH

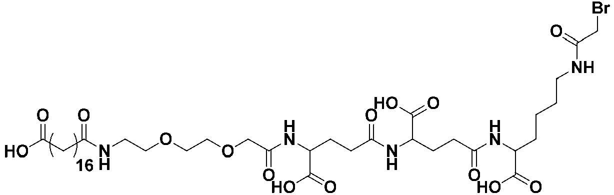

### (1) Materials and reagents

### 2-CTC resin with a substitution value of 1.15 mmol/g

Amino acids: Fmoc-Lys(Alloc)-OH, Fmoc-AEEA-OH, Fmoc-Glu-otBu, monoterbutyl octadecanedioate

### Synthetic reagents: HOBt, DIC, DMF, DCM, PIP, DIEA.

### (2) Instruments

CS-BIO peptide synthesizer, Waters600 semi-preparative high-performance liquid chromatography instrument, Beckman centrifuge, BUCHI rotary evaporator.

### (3) Synthesis of brominated fatty acid side chains

### a. Solid-phase chemical synthesis of polypeptides

1.00 g of 2-CTC resin was weighed and placed in the reactor of the peptide synthesizer, 10 ml of DCM was added, and the resin was allowed to swell for 1 hour. 2-3 times the amount of Fmoc-Lys(Alloc)-OH were weighed, 4-6 times the amount of DIEA were taken, 10 ml of DMF was added to dissolve them, the mixture was put into the reactor, and reacted at room temperature for 2 hours. That is, the first amino acid was coupled to the resin. Then, the resin was washed with DCM for 6 times and the substitution degree (SD) of the resin was determined at this time. Subsequently, 10 ml of 20% PIP/DMF solution was added, mixed for 10 minutes to remove the amino-protecting group Fmoc, this process was repeated once, then the resin was washed with DCM for 6 times. For coupling the second amino acid, three times the amount of Fmoc-Glu-otBu, HOBt, and DIC were weighted, 10 ml of DMF/DCM (1:1) mixed solvent was added to dissolve them, and reacted at room temperature. The reaction progress was monitored with ninhydrin; a colorless result indicated the completion of the reaction. The resin was washed with DCM for 6 times. And then, the coupling reactions of AEEA, glutamic acid, and octadecanedioic acid was continued according to the above coupling method, and this cycle was repeated until all amino acids were coupled completely.

Secondly, the Alloc protecting group on the side chain of Fmoc-Lys(Alloc)-OH was selectively removed using the orthogonal protection method. The specific method for removing the Alloc protecting group was as follows: palladium tetrakis(triphenylphosphine) (0.1 equivalent) and phenylsilane (10 equivalents) were weighed, dissolved in 15 ml of DCM, and added into the reactor for reacting with the resin-bound peptide for 25 minutes. This entire process must be carried out in darkness and under nitrogen atmosphere. After the reaction was completed, the resin was sequentially washed with DCM (15 ml × 6 times, 2 minutes each time), 0.02 mol/L N,N-diethyldithiocarbamate/DMF solution (15 ml × 3 times, 2 minutes each time), and DMF (15 ml × 6 times, 2 minutes each time). Then a small amount of resin was taken for ninhydrin detection. If the resin particles turned purple-black, it indicates that the deprotection was complete.

Finally, 2 equivalents of bromoacetyl bromide and 10 ml of DMF were added and reacted at room temperature for 1 hour. The reaction progress was monitored with ninhydrin. The reaction was complete when it turned colorless. The resin was washed with DCM for 6 times, the resin peptide was dried *in vacuo* for future use.

### b. Cleavage and precipitation

The cleavage reagent was added at a ratio of 10 ml cleavage reagent per 1 g of resin. The reagent ratio was TFA: TIS: H₂O = 95: 2.5: 2.5 (V:V). The reaction was conducted at room temperature for 1 hour, the resin was filtered off, and the filtrate was evaporated *in vacuo* at 40°C to remove as much TFA as possible. The fatty acid side chain was precipitated with 7-10 times the volume of ice-cold diethyl ether, placed in a -20°C refrigerator for 20 minutes, centrifuged, and dried *in vacuo* to obtain the crude fatty acid side chain product.

Purity: 66%; theoretical molecular weight: 966.96 Da; actual molecular weight was verified to be correct by LC-MS.

### 2.2 Synthesis of C18 Dicarboxylic Acid-gGlu-AEEA-AEEA-Lys(bromoacetyl)-COOH

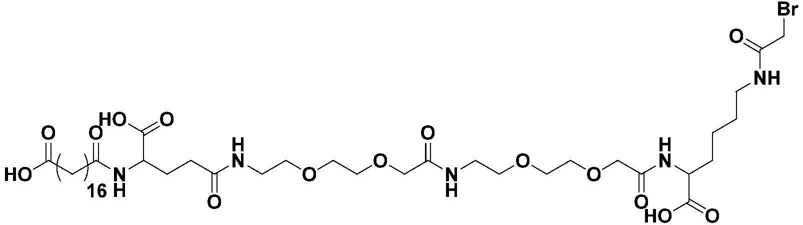

Synthesis was conducted according to 2.1 and conventional solid-phase synthesis methods.

Purity: 57%; theoretical molecular weight: 983.01 Da; actual molecular weight was verified to be correct by LC-MS.

### 2.3 Synthesis of C20 Dicarboxylic Acid-AEEA-gGlu-gGlu-Lys(bromoacetyl)-COOH

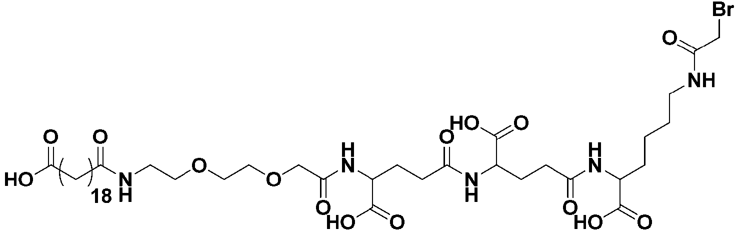

Synthesis was conducted according to 2.1 and conventional solid-phase synthesis methods.

Purity: 44%; theoretical molecular weight: 995.02 Da; actual molecular weight was verified to be correct by LC-MS.

### 2.4 Synthesis of C20 Dicarboxylic Acid-gGlu-AEEA-AEEA-Lys(bromoacetyl)-COOH

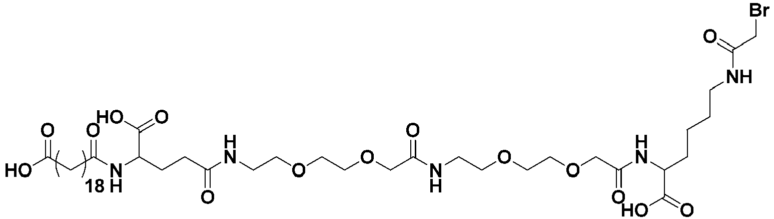

Synthesis was conducted according to 2.1 and conventional solid-phase synthesis methods.

Purity: 59%; theoretical molecular weight: 1011.06 Da; actual molecular weight was verified to be correct by LC-MS.

### 2.5 C18 Dicarboxylic Acid-gGlu-AEEA-AEEA-Lys(3,5-bis[(2-bromoacetyl)amino]benzoyl)-COOH

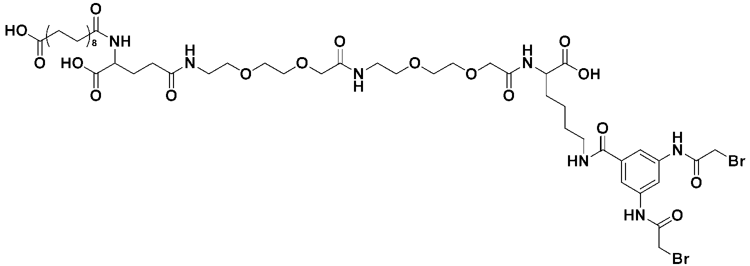

First, (2,5-dioxopyrrolidin-1-yl)-3,5-bis[(2-bromoacetyl)amino]benzoate was synthesized in accordance with CN112839681A. C18 Dicarboxylic Acid-gGlu-AEEA-AEEA-Lys-COOH was synthesized according to section 2.2 and conventional solid-phase synthesis methods, and dissolved in 2% triethylamine. (2,5-dioxopyrrolidin-1-yl)-3,5-bis[(2-bromoacetyl)amino]benzoate was dissolved in ethanol and added dropwise to C18 Dicarboxylic Acid-gGlu-AEEA-AEEA-Lys-COOH at a molar ratio of 1:1.2. After the reaction was conducted for 20-30 minutes, acetic acid was added to adjust the pH to 5.0, evaporated *in vacuo* at 37°C until a solid precipitated, and water was added to obtain a resulting white solid as the desired product.

Purity: 59%; theoretical molecular weight: 1238 Da; actual molecular weight was verified to be correct by LC-MS.

### Example 3: Determination of in vitro activities of analogs by dual-luciferase reporter gene assay

iLite FGF21 assay ready cells (purchased from SAVR) is a genetically engineered reporter gene cell line that responds to signals downstream to FGF21 agonism through the expression of firefly luciferase. The receptor FGFR1c and the cofactor β-Klotho were co-overexpressed on the surface of iLite FGF21 assay ready cells and have undergone genetic optimization to enhance sensitivity, such that FGF21 will cause an increase in luciferase expression in a dose-response manner. The luciferase activity detection kit was purchased from Promega (catalog number #E2980).

iLite FGF21 assay ready cells were cultured adherently in basal medium (DMEM Gluta Max, purchased from Gibco) containing 10% fetal bovine serum (FBS, purchased from Gibco) and 1% penicillin/streptomycin (purchased from Gibco). For the activity determination experiment, 1-30,000 cells per well and test samples such as FGF21 mutant proteins and FGF21 conjugate molecules modified with albumin-binding agent were added to all-white 96-well culture plates (purchased from Corning), and the chemiluminescence values were detected in a microplate reader according to the instructions of the luciferase activity detection kit. Finally, the half-maximal effective concentration (EC₅₀) values of the tested samples were obtained by means of four-parameter fitting.

### Example 4: In vitro activity of FGF21 mutants

Cysteine mutations were performed on Q28 (SEQ ID NO: 4), E30 (SEQ ID NO: 5), D38 (SEQ ID NO: 6), D46 (SEQ ID NO: 7), K122 (SEQ ID NO: 8), and H125 (SEQ ID NO: 9) of FGF21RGE. Construction of engineering strains, fermentation, and purification of these FGF21 cysteine mutants were performed as described in Example 1. Except for the FGF21Q28C mutant, which was difficult to be expressed and purified, all other mutants were successfully prepared.

| **Protein or mutant** | **Theoretical molecular weight/Da** | **Da Actual molecular weight/Da** |
|---|---|---|
| FGF21RGE (SEQ ID NO: 2) | 19453.8 | 19453.0 |
| FGF21Q28C (SEQ ID NO: 4) | 19427.8 | - |
| FGF21E30C (SEQ ID NO: 5) | 19427.8 | 19428.0 |
| FGF21D38C (SEQ ID NO: 6) | 19441.9 | 19442.1 |
| FGF21D46C (SEQ ID NO: 7) | 19441.9 | 19441.3 |
| FGF21K122C (SEQ ID NO: 8) | 19428.8 | 19428.3 |
| FGF21H125C (SEQ ID NO: 9) | 19419.8 | 19419.4 |

The *in vitro* activities of FGF21RGE and FGF21 cysteine mutants were evaluated using the method described in Example 3. The results are shown in the table below. All successfully expressed FGF21 mutants exhibited activities in activating the FGFR1c receptor to transmit downstream signals. Compared with the FGF21RGE control protein, the activity of FGF21Q28C decreased significantly, while the other mutants showed *in vitro* activity comparable to that of the control protein.

| **Protein or mutant** | **EC₅₀(nM)** |
|---|---|
| FGF21RGE (SEQ ID NO: 2) | 0.25 |
| FGF21Q28C (SEQ ID NO: 4) | - |
| FGF21E30C (SEQ ID NO: 5) | 1.31 |
| FGF21D38C (SEQ ID NO: 6) | 1.00 |
| FGF21D46C (SEQ ID NO: 7) | 0.28 |
| FGF21K122C (SEQ ID NO: 8) | 0.30 |
| FGF21H125C (SEQ ID NO: 9) | 0.28 |

### Example 5: Preparation of side chain-modified FGF21 analogs

The preparation process of the representative compound is given by compound 5.1.

### Preparation of 5.1 (Compound 5.1)

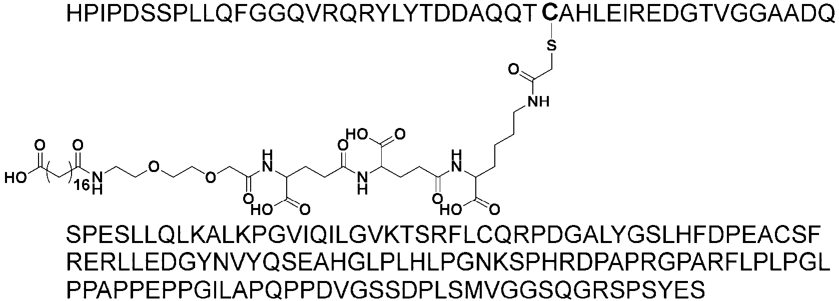

This compound is an FGF21 analog modified by the albumin-binding agent as described in Example 2.1 from the FGF21E30C mutant (Example 4) as shown in SEQ NO: 5. The specific preparation process and identification are as follows.

Before the modification reaction, the FGF21E30C mutant was ultrafiltered into Tris-HCl buffer (pH 8.0) to a final concentration of 0.5-2 mg/ml. Meanwhile, 0.2 mM TCEP (tris(2-carboxyethyl)phosphine, dissolved in Tris-HCl buffer and adjusted to pH 8.0) and 2-2.5 mM EDTA·2Na were added. The side chain as described in Example 2.1 was dissolved in a 5 M potassium iodide solution (dissolved in Tris-HCl buffer and adjusted to pH 8.0) to a concentration of 4-10 mg/ml. The dissolved albumin-binding agent solution was added to the FGF21E30C mutant at a molar ratio of FGF21E30C mutant to albumin-binding agent of 1:3, and the reaction was carried out overnight under stirring conditions. After the reaction was complete, the FGF21 analog modified by the albumin-binding agent was purified by anion exchange. Purified water was added to the reaction solution to adjust the conductivity to 6 mS/cm, and the sample was loaded onto a pre-equilibrated HiTrap Q HP (purchased from Cytiva). Mobile phase A was 25 mM Tris-HCl (pH 8.0), and mobile phase B was 25 mM Tris-HCl with 1 M NaCl (pH 8.0), with a flow rate of 5 ml/min and a gradient of 0-50% over a 40-minute run time. The target peaks were combined after analysis by RP-HPLC and SDS-PAGE to obtain the FGF21 analog with high purity, which is the FGF21E30C mutant of SEQ NO: 4 (Example 4) modified by the albumin-binding agent as described in Example 2.1.

Purity: >90%; theoretical molecular weight: 20313.9 Da; actual molecular weight: 20313.8 Da.

### Preparation of 5.2 (Compound 5.2)

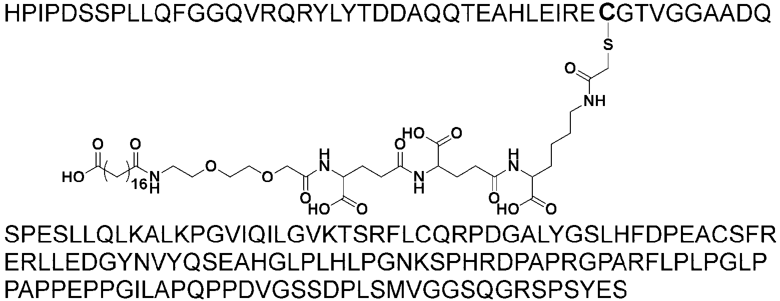

This compound is an FGF21 analog modified by the albumin-binding agent as described in Example 2.1 from the FGF21D38C mutant of SEQ NO: 6 (Example 4). The preparation process and identification are as follows.

Process: Prepared as described for Compound 5.1.

Purity: >90%; theoretical molecular weight: 20328 Da; actual molecular weight: 20327.6 Da.

### Preparation of 5.3 (Compound 5.3)

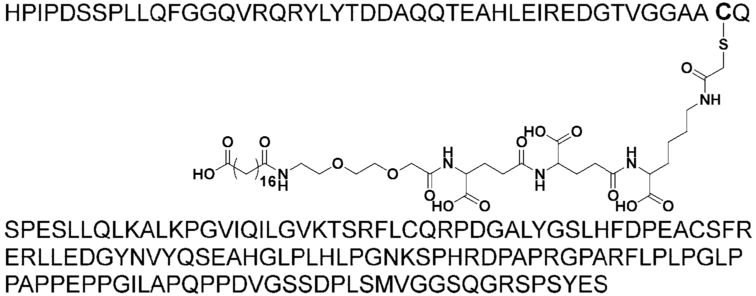

This compound is an FGF21 analog modified by the albumin-binding agent as described in Example 2.1 from the FGF21D46C mutant of SEQ NO: 7 (Example 4). The preparation process and identification are as follows.

Process: Prepared as described for Compound 5.1.

Purity: >90%; theoretical molecular weight: 20328 Da; actual molecular weight: 20327.6 Da.

### Preparation of 5.4 (Compound 5.4)

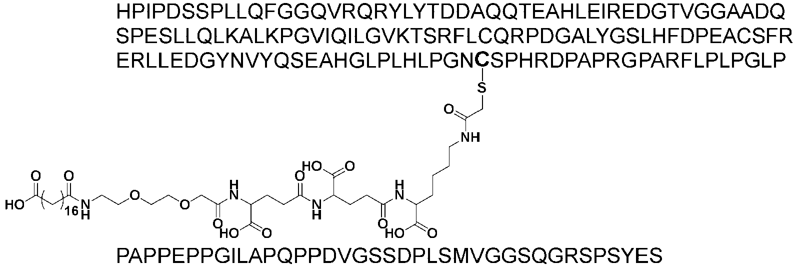

This compound is an FGF21 analog modified by the albumin-binding agent as described in Example 2.1 from the FGF21K122C mutant of SEQ NO: 8 (Example 4). The preparation process and identification are as follows.

Process: Prepared as described for Compound 5.1.

Purity: >90%; theoretical molecular weight: 20314.9 Da; actual molecular weight: 20314.7 Da.

### Preparation of 5.5 (Compound 5.5)

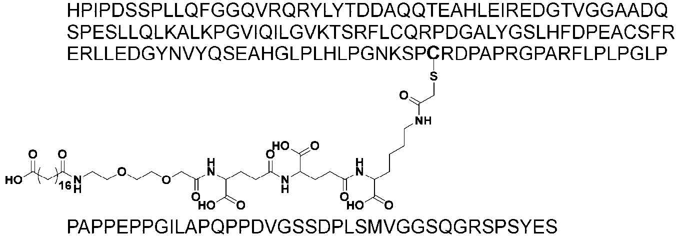

This compound is an FGF21 analog modified by the albumin-binding agent as described in Example 2.1 from the FGF21H125C mutant of SEQ NO: 9 (Example 4). The preparation process and identification are as follows.

Process: Prepared as described for Compound 5.1.

Purity: >90%; theoretical molecular weight: 20305.9 Da; actual molecular weight: 20306.6 Da.

### Preparation of 5.6 (Compound 5.6)

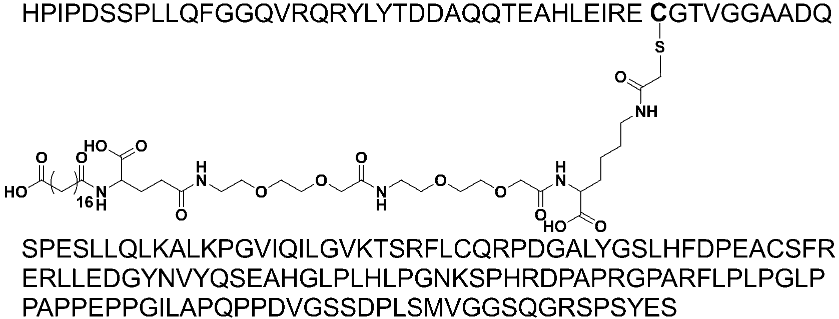

This compound is an FGF21 analog modified by the albumin-binding agent as described in Example 2.2 from the FGF21D38C mutant of SEQ NO: 6 (Example 4). The preparation process and identification are as follows.

Process: Prepared as described for Compound 5.1.

Purity: >90%; theoretical molecular weight: 20344 Da; actual molecular weight: 20343.5 Da.

### Preparation of 5.7 (Compound 5.7)

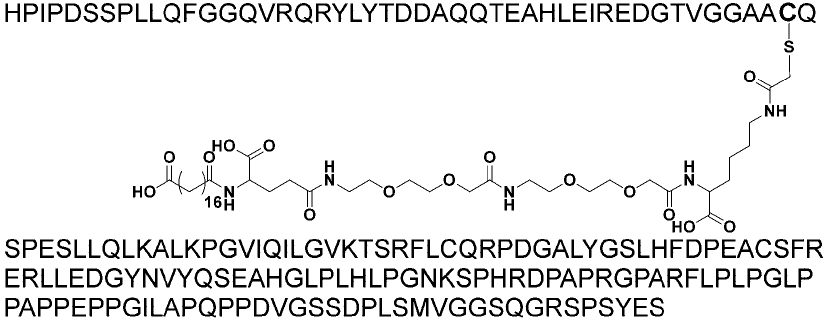

This compound is an FGF21 analog modified by the albumin-binding agent as described in Example 2.2 from the FGF21D46C mutant of SEQ NO: 7 (Example 4). The preparation process and identification are as follows.

Process: Prepared as described for Compound 5.1.

Purity: >90%; theoretical molecular weight: 20344 Da; actual molecular weight: 20343.1 Da.

### Preparation of 5.8 (Compound 5.8)

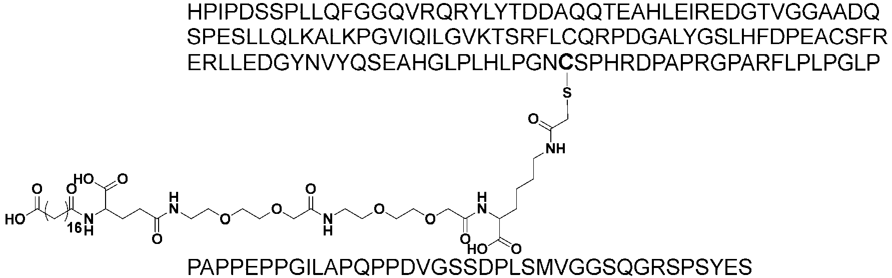

This compound is an FGF21 analog modified by the albumin-binding agent as described in Example 2.2 from the FGF21K122C mutant of SEQ NO: 8 (Example 4). The preparation process and identification are as follows.

Process: Prepared as described for Compound 5.1.

Purity: >90%; theoretical molecular weight: 20330.9 Da; actual molecular weight: 20330.3 Da.

### Preparation of 5.9 (Compound 5.9)

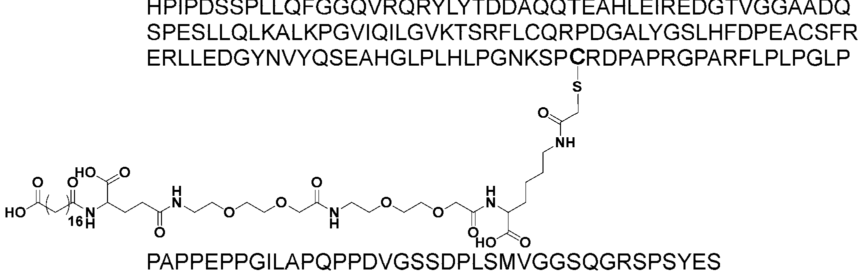

This compound is an FGF21 analog modified by the albumin-binding agent as described in Example 2.2 from the FGF21H125C mutant of SEQ NO: 9 (Example 4). The preparation process and identification are as follows.

Process: Prepared as described for Compound 5.1.

Purity: >90%; theoretical molecular weight: 20321.9 Da; actual molecular weight: 20321.4 Da.

### Preparation of 5.10 (Compound 5.10)

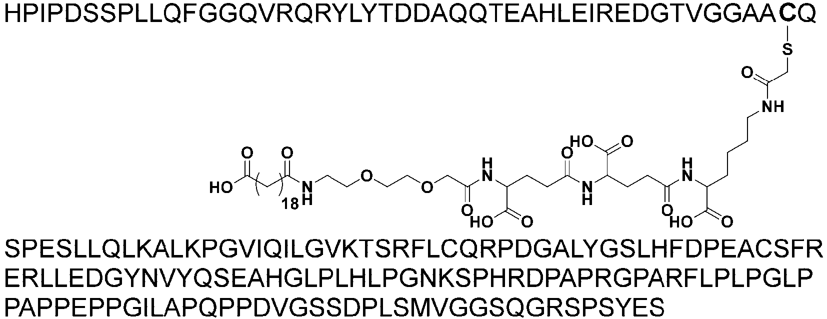

This compound is an FGF21 analog modified by the albumin-binding agent as described in Example 2.3 from the FGF21D46C mutant of SEQ NO: 7 (Example 4). The preparation process and identification are as follows.

Process: Prepared as described for Compound 5.1.

Purity: >90%; theoretical molecular weight: 20356 Da; actual molecular weight: 20355.2 Da.

### Preparation of 5.11 (Compound 5.11)

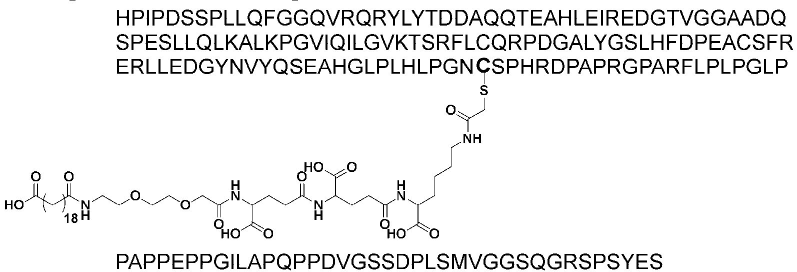

This compound is an FGF21 analog modified by the albumin-binding agent as described in Example 2.3 from the FGF21K122C mutant of SEQ NO: 8 (Example 4). The preparation process and identification are as follows.

Process: Prepared as described for Compound 5.1.

Purity: >90%; theoretical molecular weight: 20342.9 Da; actual molecular weight: 20342.3 Da.

### Preparation of 5.12 (Compound 5.12)

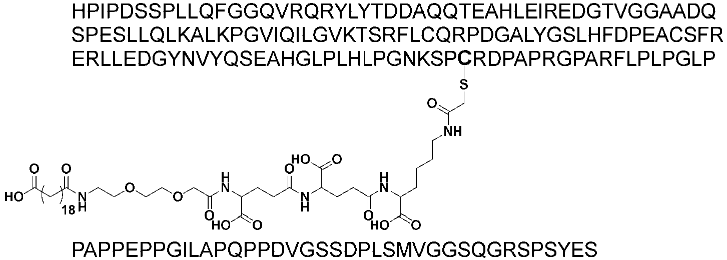

This compound is an FGF21 analog modified by the albumin-binding agent as described in Example 2.3 from the FGF21H125C mutant of SEQ NO: 9 (Example 4). The preparation process and identification are as follows.

Process: Prepared as described for Compound 5.1.

Purity: >90%; theoretical molecular weight: 20333.9 Da; actual molecular weight: 20333.1 Da.

### Preparation of 5.13 (Compound 5.13)

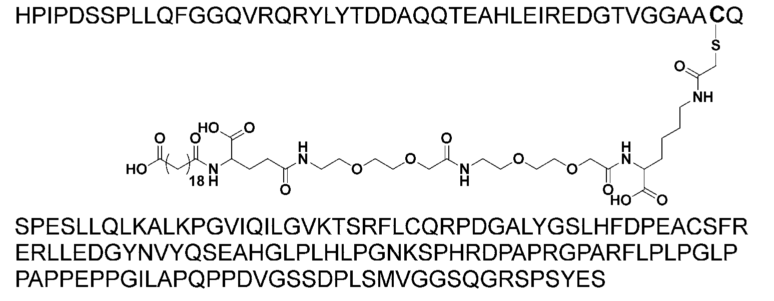

This compound is an FGF21 analog modified by the albumin-binding agent as described in Example 2.4 from the FGF21D46C mutant of SEQ NO: 7 (Example 4). The preparation process and identification are as follows.

Process: Prepared as described for Compound 5.1.

Purity: >90%; theoretical molecular weight: 20372 Da; actual molecular weight: 20370.9 Da.

### Preparation of 5.14 (Compound 5.14)

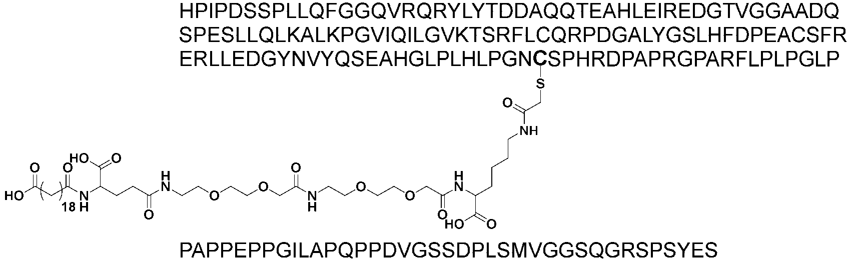

This compound is an FGF21 analog modified by the albumin-binding agent as described in Example 2.4 from the FGF21K122C mutant of SEQ NO: 8 (Example 4). The preparation process and identification are as follows.

Process: Prepared as described for Compound 5.1.

Purity: >90%; theoretical molecular weight: 20358.9 Da; actual molecular weight: 20358.1 Da.

### Preparation of 5.15 (Compound 5.15)

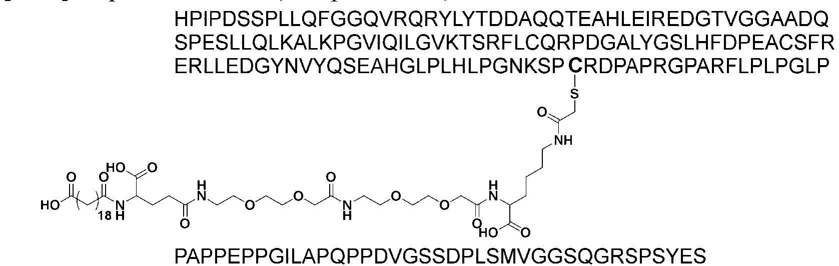

This compound is an FGF21 analog modified by the albumin-binding agent as described in Example 2.4 from the FGF21H125C mutant of SEQ NO: 9 (Example 4). The preparation process and identification are as follows.

Process: Prepared as described for Compound 5.1.

Purity: >90%; theoretical molecular weight: 20349.9 Da; actual molecular weight: 20349.3 Da.

### Example 6: In vitro activity of FGF21 analogs modified by albumin-binding agent

*In vitro* activities of FGF21RGE, FGF21 cysteine mutants, and FGF21 analogs modified by albumin-binding agent were evaluated using the method of Example 3. The results are shown in the table below.

| **Protein, mutant, or conjugate** | **EC₅₀(nM)** |
|---|---|
| FGF21RGE | 0.25 |
| FGF21E30C | 1.31 |
| FGF21D38C | 1.00 |
| FGF21D46C | 0.28 |
| FGF21K122C | 0.30 |
| FGF21H125C | 0.28 |
| compound 5.1 | 4.61 |
| compound 5.2 | 1.99 |
| compound 5.3 | 0.24 |
| compound 5.4 | 0.99 |
| compound 5.5 | 0.52 |
| compound 5.6 | 2.06 |
| compound 5.7 | 1.06 |
| compound 5.8 | 1.13 |
| compound 5.9 | 1.18 |
| compound 5.10 | 2.69 |
| compound 5.11 | 2.09 |
| compound 5.12 | 1.40 |
| compound 5.13 | 1.84 |
| compound 5.14 | 1.02 |
| compound 5.15 | 0.47 |

### Example 7: Preparation of FGF21 analogs with PAS₅₀ and side chain modifications

### 7.1 Preparation of FGF21 Mutant with a PAS₅₀ Modification (PAS₅₀FGF21)

The FGF21 fusion protein with a PAS₅₀ modification (PAS₅₀FGF21) as shown in SEQ ID NO: 10 was expressed in the bacterial expression system *E. coli* BL21(DE3) according to the method described in Example 1.

After LC-MS analysis, the actual molecular weight of PAS₅₀FGF21 prepared according to the above method is 23556.9 Da, which is consistent with the theoretical molecular weight (23557.3 Da).

### Preparation of 7.2 (Compound 7.2)

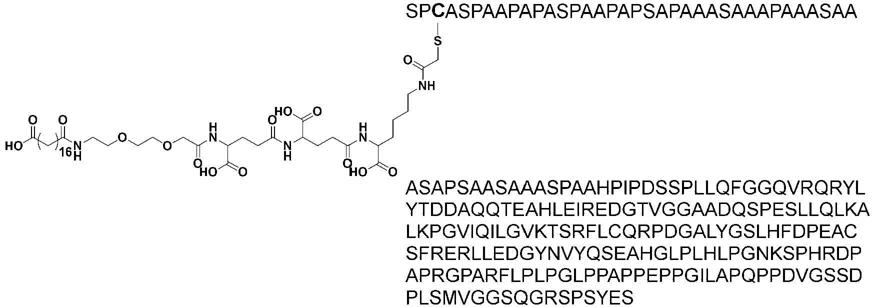

This compound is an FGF21 analog modified by the albumin-binding agent as described in Example 2.1 from the FGF21 fusion protein PAS₅₀FGF21 with a PAS₅₀ modification as shown in SEQ ID NO: 10 (Compound 7.1). The preparation process and identification are as follows.

### Process: Prepared as described for Compound 5.1.

Purity: >90%; theoretical molecular weight: 24443.4 Da; actual molecular weight: 24442.7 Da.

### Preparation of 7.3 (Compound 7.3)

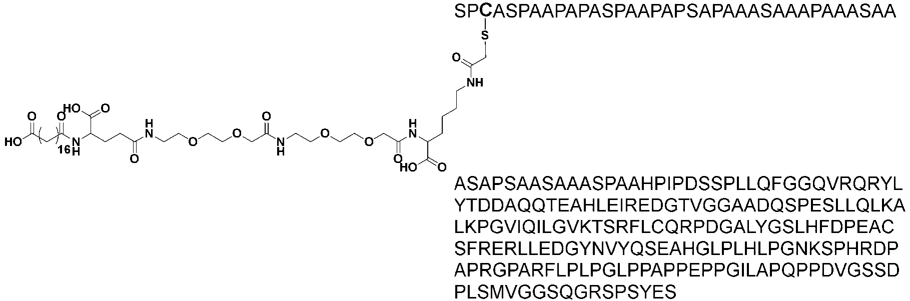

This compound is an FGF21 analog modified by the albumin-binding agent as described in Example 2.2 from the FGF21 fusion protein PAS₅₀FGF21 with a PAS₅₀ modification as shown in SEQ ID NO: 10 (Example 7.1). The preparation process and identification are as follows.

### Process: Prepared as described for Compound 5.1.

Purity: >90%; theoretical molecular weight: 24459.4 Da; actual molecular weight: 24458.9 Da.

### Preparation of 7.4 (Compound 7.4)

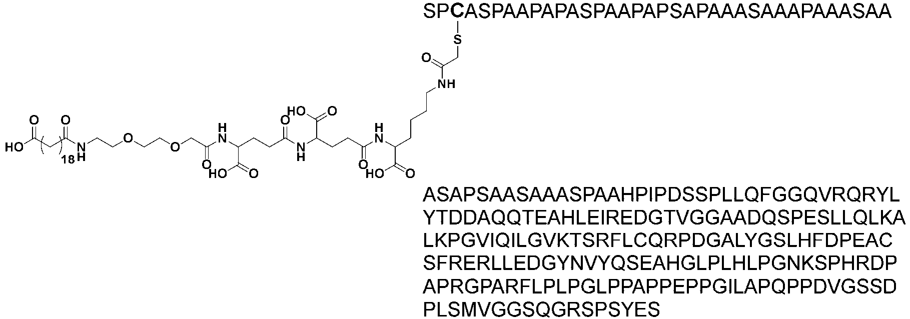

This compound is an FGF21 analog modified by the albumin-binding agent as described in Example 2.3 from the FGF21 fusion protein PAS₅₀FGF21 with a PAS₅₀ modification as shown in SEQ ID NO: 10 (Example 7.1). The preparation process and identification are as follows.

### Process: Prepared as described for Compound 5.1.

Purity: >90%; theoretical molecular weight: 24471.4 Da; actual molecular weight: 24470.9 Da.

### Preparation of 7.5 (Compound 7.5)

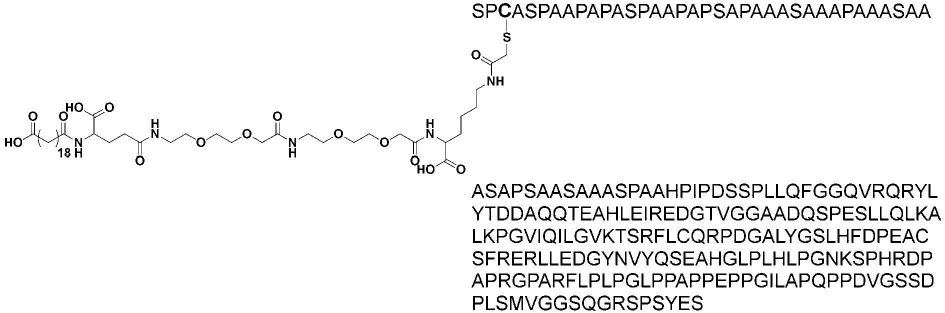

This compound is an FGF21 analog modified by the albumin-binding agent as described in Example 2.4 from the FGF21 fusion protein PAS₅₀FGF21 with a PAS₅₀ modification as shown in SEQ ID NO: 10 (Example 7.1). The preparation process and identification are as follows.

### Process: Prepared as described for Compound 5.1.

Purity: >90%; theoretical molecular weight: 24487.4 Da; actual molecular weight: 24486.9 Da.

### Example 8: Synthesis of other modifying side chains

### 8.1 Synthesis of C18 Dicarboxylic Acid-gGlu-AEEA-AEEA-Lys-OH

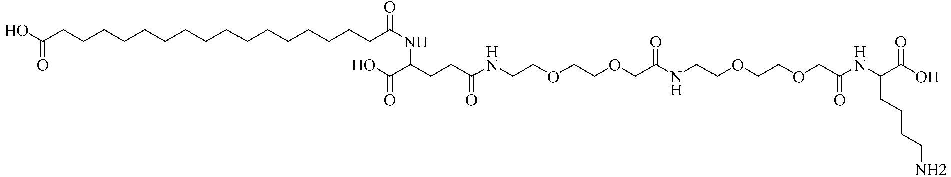

### (1) Materials and reagents

Fmoc-Lys(BOC)-Wang resin with a substitution value of 0.5 mmol/g.
Amino acids: Fmoc-AEEA-OH, Fmoc-Glu-otBu, monoterbutyl octadecanedioate
Synthetic reagents: HOBt, DIC, DMF, DCM, PIP, DIEA.

### (2) Instruments

CS-BIO polypeptide synthesizer, Waters600 semi-preparative high-performance liquid chromatograph, Beckman centrifuge, BUCHI rotary evaporator.

### (3) Synthesis of brominated fatty acid side chains

### a. Solid-phase chemical synthesis of polypeptides

1.00 g of Fmoc-Lys(BOC)-Wang resin was weighed and placed in the reactor of the peptide synthesizer, 10 ml od DCM was added, and the resin was allowed to swell for 1 hour. Then, 10 ml of 20% PIP/DMF solution was added and mixed for 10 minutes to remove the amino-protecting group Fmoc, and this process was repeated once. Subsequently, the resin was washed with DCM for 6 times. For coupling the second amino acid, Fmoc-AEEA-OH, HOBt, and DIC in an amount three times the weight of the resin was weighed, dissolved in 10 ml of DMF/DCM (1:1) mixed solvent, and reacted at room temperature. The reaction progress was monitored using ninhydrin; a colorless result indicates the completion of the reaction. The resin was washed with DCM for 6 times. Then, the coupling reactions of AEEA, glutamic acid, and octadecanedioic acid was continued according to the above coupling method, and this cycle was repeated until all amino acids were coupled completely.

### b. Cleavage and precipitation

The cleavage reagent was added at a ratio of 10 ml cleavage reagent per 1 g of resin. The reagent ratio was TFA: TIS: H₂O = 95: 2.5: 2.5 (V:V). The reaction was conducted at room temperature for 1 hour, and the resin was filtered off. The fatty acid side chain was precipitated with 7-10 times the volume of ice-cold diethyl ether, placed in a -20°C refrigerator for 20 minutes, centrifuged, and dried *in vacuo* to obtain the crude peptide.

Purity: 90%; theoretical molecular weight: 862.07 Da; the actual molecular weight was verified to be correct by LC-MS.

### 8.2 Synthesis of C18 Dicarboxylic Acid-gGlu-AEEA-AEEA-Lys-NH₂

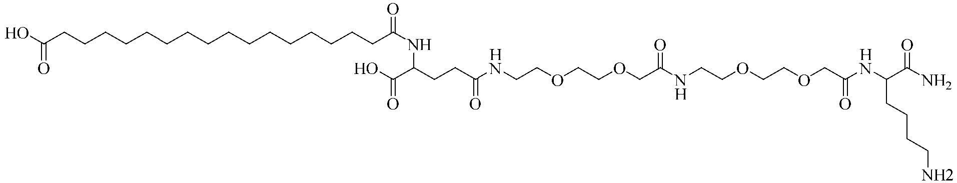

### (1) Materials and reagents

Rink Amide MBHA resin with a substitution value of 0.5 mmol/g.
Amino acids: Fmoc-Lys(BOC)-OH, Fmoc-AEEA-OH, Fmoc-Glu-otBu, monotert-butyl octadecanedioate
Synthetic reagents: HOBt, DIC, DMF, DCM, PIP, DIEA.
(2) Synthesis was performed according to the solid-phase synthesis method described in 8.1;
Purity: 91%; theoretical molecular weight: 861.07 Da; the actual molecular weight was verified to be correct by LC-MS.

### 8.3 Synthesis of 3,5-bis[(2-bromoacetyl)amino]benzoic acid

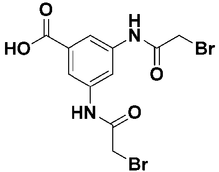

Diaminobenzoic acid (2 g, 0.013 mol) was added to 40 ml of DMF (20 times the solvent) for dissolution. Under ice bath conditions, 5.81 ml of bromoacetyl bromide (2.2 eq) was added, and the reaction was carried out at room temperature for 3.5 hours. The reaction solution was added to 20 times the volume of water, placed at 4°C overnight, suction-filtered, washed with water for 3 times, and freeze-dried to obtain 3 g of intermediate powder.

Purity: 87%; theoretical molecular weight: 394 Da; the actual molecular weight was verified to be correct by LC-MS.

### 8.4 Synthesis of (2,5-dioxopyrrolidin-1-yl)-3,5-bis[(2-bromoacetyl)amino]benzoate

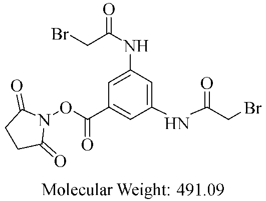

The intermediate powder (3 g, 7.6 mmol) obtained in Example 8.3 was dissolved in 60 ml of THF. HOSU (1.31 g, 11.4 mmol, 1.5 eq) and DCC (2.35 g, 11.4 mmol, 1.5 eq) were added, and the mixture was activated at room temperature for 1 hour, filtered to remove the by-product DCU, and distilled under a reduced pressure to remove the THF. The residue was redissolved in ethyl acetate, filtered to remove insoluble DCU, distilled under a reduced pressure to remove the ethyl acetate and vacuum-dried for 24 hours to give 4.76 g of a dark yellow solid powder.

Purity: 83%; theoretical molecular weight: 491.09 Da; the actual molecular weight was verified to be correct by LC-MS.

### 8.5 Synthesis of C18 Dicarboxylic Acid-gGlu-AEEA-AEEA-Lys(3,5-bis[(2-bromoacetyl)amino]benzoyl)-OH

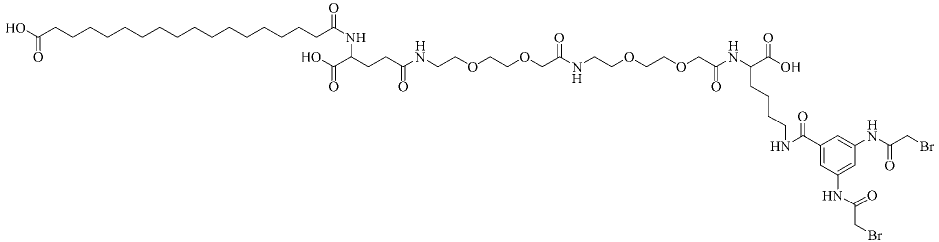

The compound prepared in Example 8.1 was dissolved in 2% triethylamine, (2,5-dioxopyrrolidin-1-yl)-3,5-bis[(2-bromoacetyl)amino]benzoate was dissolved in ethanol, and added dropwise to the crude peptide solution at a molar ratio of 1: 1.2. After reacting for 1-3 hours, acetic acid was added to adjust the pH to 6.6, evaporated *in vacuo* until a solid precipitated, and water was added to obtain a white solid as the desired product.

Purity: 52%; theoretical molecular weight: 1238 Da; the actual molecular weight was verified to be correct by LC-MS.

### 8.6 Synthesis of C18 Dicarboxylic Acid-gGlu-AEEA-AEEA-Lys(3,5 Bis[(2 bromoacetyl)amino]benzoyl)-NH₂

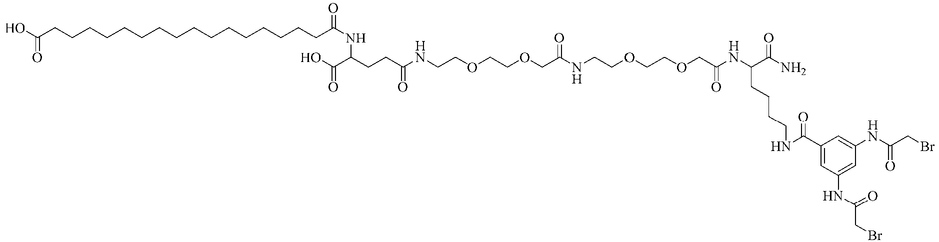

The crude peptide in Example 8.2 was dissolved in 2% triethylamine. (2,5-dioxopyrrolidin-1-yl) 3,5-bis[(2-bromoacetyl)amino]benzoate was dissolved in ethanol and added dropwise to the crude peptide solution at a molar ratio of 1:1.2. After reacting for 1-3 hours, acetic acid was added to adjust the pH to 6.6. The mixture was evaporated *in vacuo* until a solid precipitated, and water was added to obtain a white solid as the desired product.

Purity: 50%; theoretical molecular weight: 1237 Da; the actual molecular weight has been verified to be correct by LC-MS.

### Example 9: In vitro activity of FGF21 mutants

The construction of the engineering strain, fermentation, and purification for these FGF21 mutants were conducted as described in Example 1. All mutants were successfully prepared.

| **Protein or mutant** | **Theoretical molecular weight/Da** | **Actual molecular weight/Da** |
|---|---|---|
| FGF21RGE(SEQ ID NO: 2) | 19453.8 | Confirmed |
| PAS₁₅FGF21(SEQ ID NO: 13) | 20803.3 | Confirmed |
| PAS₃₀FGF21(SEQ ID NO: 14) | 22005.7 | Confirmed |
| PAS₅₀FGF21(SEQ ID NO: 15) | 23557.3 | Confirmed |
| PAS₁₀₀FGF21(SEQ ID NO: 16) | 27671.8 | Confirmed |
| PAS₂₀₀FGF21(SEQ ID NO: 17) | 35682.6 | Confirmed |

The *in vitro* activity of FGF21RGE and PAS-modified FGF21 cysteine mutants was evaluated using the method described in Example 3. The results are shown in the table below. All successfully expressed FGF21 mutants exhibited activity in activating the FGFR1c receptor to transmit downstream signals. Compared with the FGF21RGE control protein, the activity of PAS₂₀₀FGF21 decreased significantly, while the other mutants showed *in vitro* activity comparable to that of the control protein.

| **Protein or mutant** | **EC₅₀(nM)** |
|---|---|
| FGF21RGE(SEQ ID NO: 2) | 0.25 |
| PAS₁₅FGF21(SEQ ID NO: 13) | 0.22 |
| PAS₃₀FGF21(SEQ ID NO: 14) | 0.30 |
| PAS₅₀FGF21 (SEQ ID NO: 15) | 0.40 |
| PAS₁₀₀FGF21(SEQ ID NO: 16) | 0.62 |
| PAS₂₀₀FGF21(SEQ ID NO: 17) | 1.98 |

### Example 10: Preparation of PAS-FGF21 Dimer Analogs

### Preparation of 10.1 (Compound 10.1)

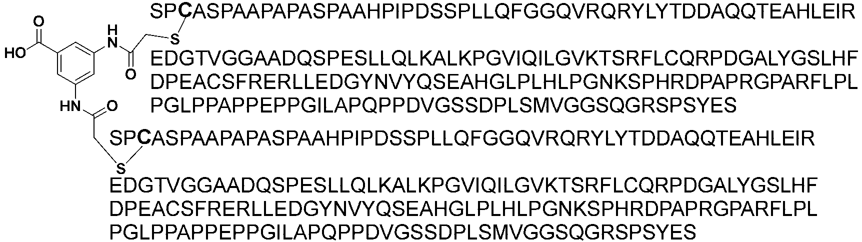

This compound is an FGF21 analog modified by the side chain as described in Example 8.3 from the FGF21 fusion protein PAS₁₅FGF21 with a PAS₁₅ modification as shown in SEQ ID NO: 13. The preparation process and identification are as follows.

Process: Before the modification reaction, the PAS₁₅FGF21 mutant was ultrafiltered into Tris-HCl, 10 mM EDTA·2Na buffer (pH 7.5) to a final concentration of 1-4 mg/ml, and added to 0.1 mM TCEP (tris(2-carboxyethyl)phosphine, dissolved in Tris-HCl buffer, adjust the pH to 8.0). The side chain as described in Example 8.3 was dissolved in ethanol (with a concentration of 4-10 mg/ml), and the molar ratio of the side chain to the protein was side chain : protein = 0.9 : 2. The side chain solution was added in ten portions at 5-minute intervals for the reaction, and reacted overnight under stirring conditions. After the reaction, the PAS-FGF21 dimer analog was purified by anion exchange. The purified water was added to the reaction solution to adjust the conductivity to 6 mS/cm, and the sample was loaded onto a pre-equilibrated HiTrap Q HP (purchased from Cytiva). Mobile phase A was 25 mM Tris-HCl (pH 8.0), mobile phase B was 25 mM Tris-HCl with 1 M NaCl (pH 8.0), the flow rate was 5 ml/min, and a 0-50% gradient was run over 40 minutes. The target peaks were combined after analysis by RP-HPLC and SDS-PAGE, resulting in an FGF21 analog modified by the albumin-binding agent as described in Example 8.3 from the PAS₁₅FGF21 mutant as shown in SEQ ID NO: 13 with a relatively high purity.

Purity: >85%; theoretical molecular weight: 41838.8 Da; actual molecular weight: confirmed.

### Preparation of 10.2 (Compound 10.2)

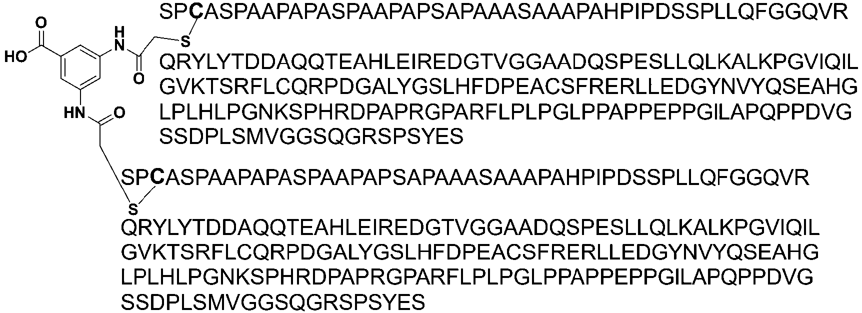

This compound is an FGF21 analog modified by the side chain as described in Example 8.3 from the FGF21 fusion protein PAS30FGF21 with a PAS50 modification as shown in SEQ ID NO: 14. The preparation process and identification are as follows.

Process: prepared as described for compound 10.1. The molar ratio of the side chain to the protein was side chain : protein = 0.9 : 2. The side chain was dissolved in ethanol, and the side chain solution was added in ten portions at 5-minute intervals for the reaction.

Purity: >85%; theoretical molecular weight: 44243.6 Da; actual molecular weight: confirmed.

### Preparation of 10.3 (Compound 10.3)

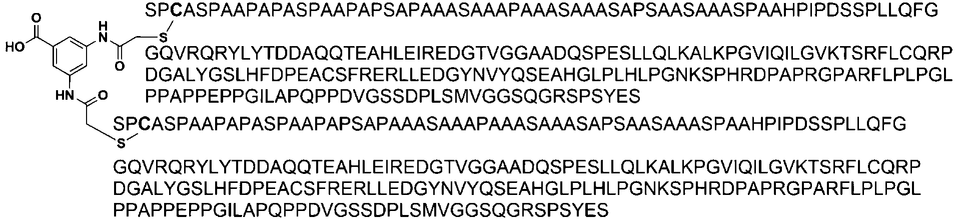

This compound is an FGF21 analog modified by the side chain as described in Example 8.3 from the FGF21 fusion protein PAS₅₀FGF21 with a PAS₅₀ modification as shown in SEQ ID NO: 15. The preparation process and identification are as follows.

Process: prepared as described for compound 10.1. The molar ratio of the side chain to the protein was side chain : protein = 0.9 : 2. The side chain was dissolved in ethanol, and the side chain solution was added in ten portions at 5-minute intervals for the reaction.

Purity: >85%; theoretical molecular weight: 47346.6 Da; actual molecular weight: confirmed.

### Preparation of 10.4 (Compound 10.4)

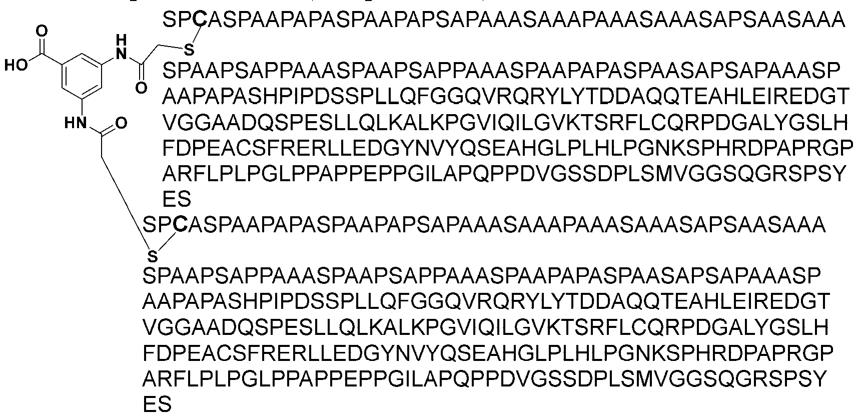

This compound is an FGF21 analog modified by the side chain as described in Example 8.3 from the FGF21 fusion protein PAS₁₀₀FGF21 with a PAS₅₀ modification as shown in SEQ ID NO: 16. The preparation process and identification are as follows.

Process: prepared as described for compound 10.1. The molar ratio of the side chain to the protein was side chain : protein = 0.9 : 2. The side chain was dissolved in ethanol, and the side chain solution was added in ten portions at 5-minute intervals for the reaction.

Purity: >85%; theoretical molecular weight: 55575.8 Da; actual molecular weight: confirmed.

### Preparation of 10.5 (Compound 10.5)

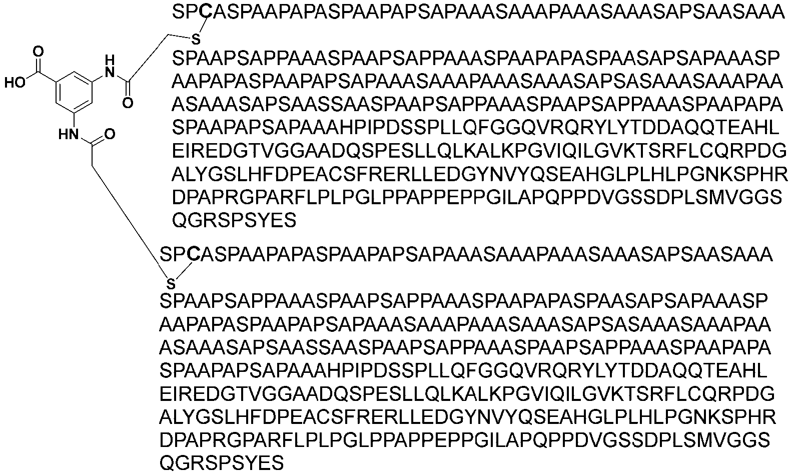

This compound is an FGF21 analog modified by the side chain as described in Example 8.3 from the FGF21 fusion protein PAS₂₀₀FGF21 with a PAS₅₀ modification as shown in SEQ ID NO: 17. The preparation process and identification are as follows.

Process: prepared as described for compound 10.1. The molar ratio of the side chain to the protein was side chain : protein = 0.9 : 2. The side chain was dissolved in ethanol, and the side chain solution was added in ten portions at 5-minute intervals for the reaction.

Purity: >85%; theoretical molecular weight: 71597.4 Da; actual molecular weight: confirmed.

### Example 11: In vitro activity of PAS-FGF21 dimer analogs

The *in vitro* activities of FGF21RGE, a series of PAS-FGF21 mutants, and PAS-FGF21 dimer analogs were evaluated using the method of Example 3. The results are shown in the following table.

| **Protein, mutant, or conjugate** | **EC₅₀(nM)** |
|---|---|
| FGF21RGE | 0.25 |
| PAS₁₅FGF21(SEQ ID NO: 13) | 0.22 |
| PAS₃₀FGF21(SEQ ID NO: 14) | 0.30 |
| PAS₅₀FGF21(SEQ ID NO: 15) | 0.40 |
| PAS₁₀₀FGF21(SEQ ID NO: 16) | 0.62 |
| PAS₂₀₀FGF21(SEQ ID NO: 17) | 1.98 |
| Compound 10.1 | 0.07 |
| Compound 10.2 | 0.04 |
| Compound 10.3 | 0.02 |
| Compound 10.4 | 0.13 |
| Compound 10.5 | 0.15 |

### Example 12: Preparation of PAS-FGF21 Dimer Analogs with Fatty Acid Modification

### Preparation of 12.1 (Compound 12.1)

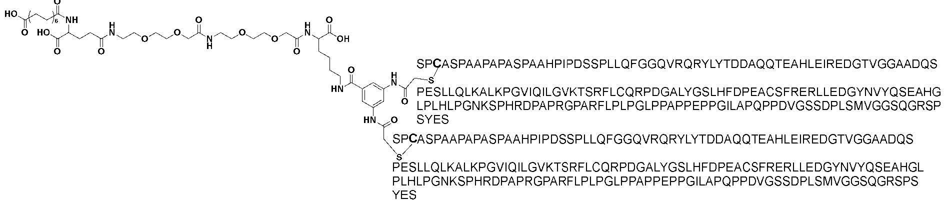

This compound is an FGF21 analog modified by the side chain as described in Example 8.5 from the FGF21 fusion protein PAS₁₅FGF21 with a PAS₁₅ modification as shown in SEQ ID NO: 13. The preparation process and identification are as follows.

### Process: prepared as described for compound 10.1.

Purity: >85%; theoretical molecular weight: 42679.8 Da; actual molecular weight: confirmed.

### Preparation of 12.2 (Compound 12.2)

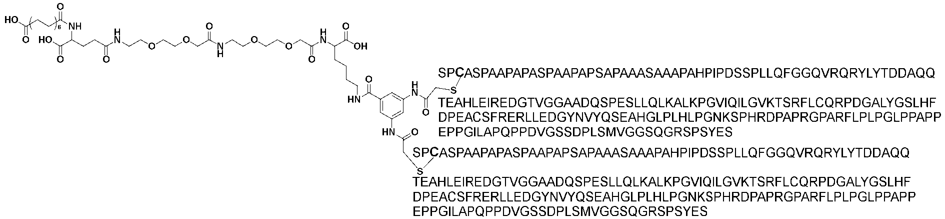

This compound is an FGF21 analog modified by the side chain as described in Example 8.5 from the FGF21 fusion protein PAS₃₀FGF21 with a PAS₃₀ modification as shown in SEQ ID NO: 14. The preparation process and identification are as follows.

Process: prepared as described for compound 10.1.

Purity: >85%; theoretical molecular weight: 45084.6 Da; actual molecular weight: confirmed.

### Preparation of 12.3 (Compound 12.3)

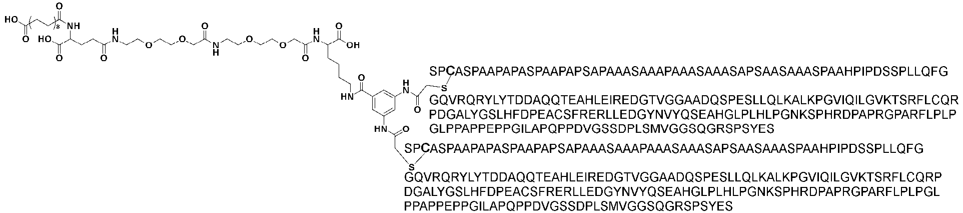

This compound is an FGF21 analog modified by the side chain as described in Example 8.5 from the FGF21 fusion protein PAS₅₀FGF21 with a PAS₅₀ modification as shown in SEQ ID NO: 15. The preparation process and identification are as follows.

Process: prepared as described for compound 10.1.

Purity: >85%; theoretical molecular weight: 48187.6 Da; actual molecular weight: confirmed.

### Preparation of 12.4 (Compound 12.4)

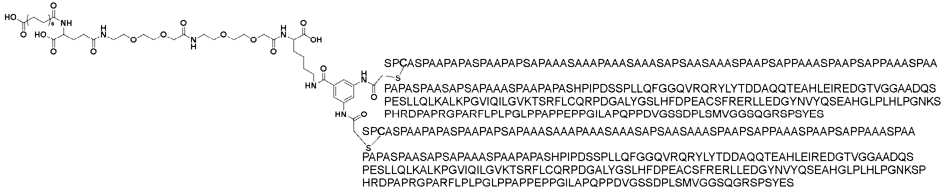

This compound is an FGF21 analog modified by the side chain as described in Example 8.5 from the FGF21 fusion protein PAS₁₀₀FGF21 with a PAS₁₀₀ modification as shown in SEQ ID NO: 16. The preparation process and identification are as follows.

Process: prepared as described for compound 10.1.

Purity: >85%; theoretical molecular weight: 56516.8 Da; actual molecular weight: confirmed.

### Preparation of 12.5 (Compound 12.5)

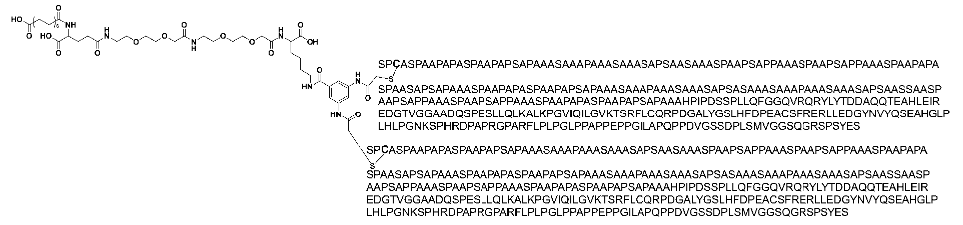

This compound is an FGF21 analog modified by the side chain as described in Example 8.5 from the FGF21 fusion protein PAS₂₀₀FGF21 with a PAS₂₀₀ modification as shown in SEQ ID NO: 17. The preparation process and identification are as follows.

Process: prepared as described for compound 10.1.

Purity: >85%; theoretical molecular weight: 72438.4 Da; actual molecular weight: confirmed.

### Preparation of 12.6 (Compound 12.6)

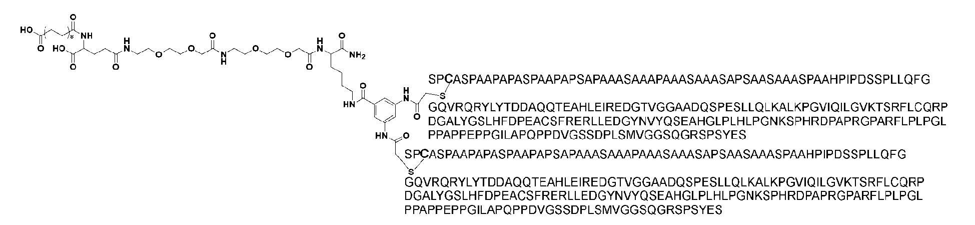

This compound is an FGF21 analog modified by the side chain as described in Example 8.6 from the FGF21 fusion protein PAS₂₀₀FGF21 with a PAS₂₀₀ modification as shown in SEQ ID NO: 17. The preparation process and identification are as follows.

Process: prepared as described for compound 10.1.

Purity: >85%; theoretical molecular weight: 48186.8 Da; actual molecular weight: confirmed.

### Example 13: In vitro activity of fatty acid-modified PAS-FGF21 dimer analogs

The *in vitro* activities of FGF21RGE, a series of PAS-FGF21 mutants, and fatty acid-modified PAS-FGF21 dimer analogs were evaluated using the method of Example 3. The results are shown in the table below.

| **Protein, mutant, or conjugate** | **EC₅₀(nM)** |
|---|---|
| FGF21RGE | 0.25 |
| PAS₁₅FGF21(SEQ ID NO: 13) | 0.22 |
| PAS₃₀FGF21(SEQ ID NO: 14) | 0.30 |
| PAS₅₀FGF21 (SEQ ID NO: 15) | 0.40 |
| PAS₁₀₀FGF21(SEQ ID NO: 16) | 0.62 |
| PAS₂₀₀FGF21(SEQ ID NO: 17) | 1.98 |
| Compound 12.1 | 0.09 |
| Compound 12.2 | 0.13 |
| Compound 12.3 | 0.11 |
| Compound 12.4 | 0.11 |
| Compound 12.5 | 0.09 |
| Compound 12.6 | - |

### Example 14: Pharmacokinetic Test

The pharmacokinetic properties of protein analogs can be tested in mice or minpigs.

Mice experiment (N = 3): The injection dose was 1 mg/kg, and the injection method was subcutaneous injection. The blood-collection time points for plasma samples were 0.08, 0.25, 0.5, 1, 2, 4, 6, 8, 12, 24, 48, and 72 hours. The blood was collected into EDTA·2K-coated test tubes (which must be temporarily stored on ice) and centrifuged at 1200 × g for 10 minutes at 4°C. Subsequently, the plasma was transferred to Micronic tubes and stored at -20°C. The human FGF21 detection ELISA kit (RD191108200R) produced by Biovendor was used to monitor the blood drug concentration.

Minipig experiment (N = 3): The injection dose was 1 mg/kg, and the injection method was subcutaneous injection. The blood-collection time points for plasma samples were 0.083, 0.25, 0.5, 1, 2, 4, 6, 8, 12, 24, 48, 72, 96, 120, 144, 168, 192, 216, 240, 264, 288 h. The blood was collected into EDTA·2K-coated test tubes (which must be temporarily stored on ice) and centrifuged at 1200 × g for 10 minutes at 4°C. Subsequently, the plasma was transferred to Micronic tubes and stored at -20°C. The human FGF21 detection ELISA kit (CAT: RD191108200R) produced by Biovendor was used to monitor the blood drug concentration.

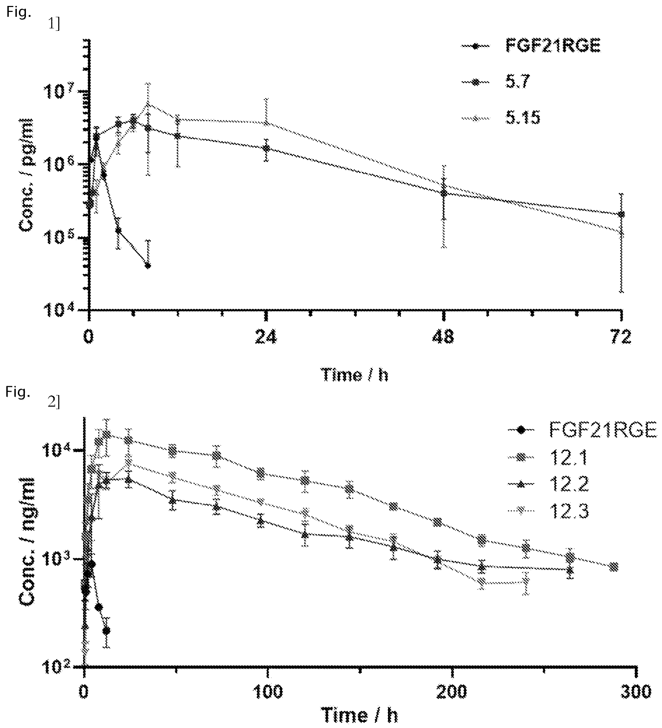

Figure 1 shows the pharmacokinetic curves in mice. The half-lives of compound 5.7 and compound 5.15 in mice are 15.9 h and 14.1 h, respectively, while the measured half-life of FGF21RGE shown in SEQ ID NO: 2 is only 0.5 h.

Figure 2 shows the pharmacokinetic curves in minipigs. The half-lives of compounds 12.2, 12.4, and 12.6 in minipigs are 65.9 ± 3.8 h, 75.4 ± 9.4 h, and 61.3 ± 1.1 h, respectively.

### Example 10 Pharmacodynamic Test in db/db Mice Induced by High-Fat Diet

Establishment of a db/db mouse model induced by high-fat diet: Male db/db mice, 8 weeks old, were adaptively fed with a standard diet for 2-3 days, after which all feed was replaced with 60% high-fat feed for rearing. After 3 weeks of high-fat feed feeding, the NASH model could be obtained. Administration: N = 6, subcutaneous injection of Vehicle and protein analogues (5 or 20 nmol/kg) was given once every three days, with an administration period of 6 weeks.

At the end of the administration, liver tissue sections were taken for hematoxylin-eosin staining to detect the effect of protein analogs on the non-alcoholic fatty liver disease activity score (NAS score) in high-fat diet-induced db/db mice.

| Groups | NAS score |
|---|---|
| Normal | 0.00 ± 0.00 |
| Vehicle | 6.00 ± 0.63 |
| FGF21RGE 5 nmol/kg | 4.83 ± 1.17 |
| FGF21RGE 20 nmol/kg | 4.17 ± 0.75 |
| Compound 12.1 5 nmol/kg | 3.83 ± 0.98 |
| Compound 12.1 20 nmol/kg | 3.33 ± 0.82 |
| Compound 12.2 5 nmol/kg | 4.33 ± 1.03 |
| Compound 12.2 20 nmol/kg | 3.40 ± 1.14 |
| Compound 12.3 5 nmol/kg | 4.33 ± 1.37 |
| Compound 12.3 20 nmol/kg | 3.00 ± 0.89 |

### Sequences involved in the examples:

SEQ ID NO: 1
   >Human mature FGF21 
HPIPDSSPLLQFGGQVRQRYLYTDDAQQTEAHLEIREDGTVGGAADQSPESLLQLKA
LKPGVIQILGVKTSRFLCQRPDGALYGSLHFDPEACSFRELLLEDGYNVYQSEAHGLPLHL
PGNKSPHRDPAPRGPARFLPLPGLPPALPEPPGILAPQPPDVGSSDPLSMVGPSQGRSPSYA
S
SEQ ID NO: 2
   >Human mature FGF21 mutant RGE(FGF21RGE) 
HPIPDSSPLLQFGGQVRQRYLYTDDAQQTEAHLEIREDGTVGGAADQSPESLLQLKA
LKPGVIQILGVKTSRFLCQRPDGALYGSLHFDPEACSFRERLLEDGYNVYQSEAHGLPLHL
PGNKSPHRDPAPRGPARFLPLPGLPPAPPEPPGILAPQPPDVGSSDPLSMVGGSQGRSPSYE
S
SEQ ID NO: 3
   >Human mature FGF21 mutant RGE comprising an N-terminal 6-histidine (His₆) tag and a small ubiquitin-like modifier (SUMO) tag (His₆-SUMO-FGF21RGE) 
MAHHHHHHSSSDSEVNQEAKPEVKPEVKPETHINLKVSDGSSEIFFKIKKTTPLRRL
MEAFAKRQGKEMDSLRFLYDGIRIQADQTPEDLDMEDNDIIEAHREQIGGHPIPDSSPLLQF
GGQVRQRYLYTDDAQQTEAHLEIREDGTVGGAADQSPESLLQLKALKPGVIQILGVKTSR
FLCQRPDGALYGSLHFDPEACSFRERLLEDGYNVYQSEAHGLPLHLPGNKSPHRDPAPRG
PARFLPLPGLPPAPPEPPGILAPQPPDVGSSDPLSMVGGSQGRSPSYES
SEQ ID NO: 4
   >Human mature FGF21 mutant Q28C(FGF21Q28C) 
HPIPDSSPLLQFGGQVRQRYLYTDDAQCTEAHLEIREDGTVGGAADQSPESLLQLKA
LKPGVIQILGVKTSRFLCQRPDGALYGSLHFDPEACSFRERLLEDGYNVYQSEAHGLPLHL
PGNKSPHRDPAPRGPARFLPLPGLPPAPPEPPGILAPQPPDVGSSDPLSMVGGSQGRSPSYE
S
 SEQ ID NO: 5
   > Human mature FGF21 mutant E30C(FGF21E30C) 
HPIPDSSPLLQFGGQVRQRYLYTDDAQQTCAHLEIREDGTVGGAADQSPESLLQLKA
LKPGVIQILGVKTSRFLCQRPDGALYGSLHFDPEACSFRERLLEDGYNVYQSEAHGLPLHL
PGNKSPHRDPAPRGPARFLPLPGLPPAPPEPPGILAPQPPDVGSSDPLSMVGGSQGRSPSYE
S
SEQ ID NO: 6
   >Human mature FGF21 mutant D38C(FGF21D38C) 
HPIPDSSPLLQFGGQVRQRYLYTDDAQQTEAHLEIRECGTVGGAADQSPESLLQLKA
LKPGVIQILGVKTSRFLCQRPDGALYGSLHFDPEACSFRERLLEDGYNVYQSEAHGLPLHL
PGNKSPHRDPAPRGPARFLPLPGLPPAPPEPPGILAPQPPDVGSSDPLSMVGGSQGRSPSYE
S
SEQ ID NO: 7
   >Human mature FGF21 mutant D46C(FGF21D46C) 
HPIPDSSPLLQFGGQVRQRYLYTDDAQQTEAHLEIREDGTVGGAACQSPESLLQLKA
LKPGVIQILGVKTSRFLCQRPDGALYGSLHFDPEACSFRERLLEDGYNVYQSEAHGLPLHL
 
PGNKSPHRDPAPRGPARFLPLPGLPPAPPEPPGILAPQPPDVGSSDPLSMVGGSQGRSPSYE
S
SEQ ID NO: 8
   >Human mature FGF21 mutant K122C(FGF21K122C) 
HPIPDSSPLLQFGGQVRQRYLYTDDAQQTEAHLEIREDGTVGGAADQSPESLLQLKA
LKPGVIQILGVKTSRFLCQRPDGALYGSLHFDPEACSFRERLLEDGYNVYQSEAHGLPLHL
PGNCSPHRDPAPRGPARFLPLPGLPPAPPEPPGILAPQPPDVGSSDPLSMVGGSQGRSPSYES
SEQ ID NO: 9
   >Human mature FGF21 mutant H125C(FGF21H125C) 
HPIPDSSPLLQFGGQVRQRYLYTDDAQQTEAHLEIREDGTVGGAADQSPESLLQLKA
LKPGVIQILGVKTSRFLCQRPDGALYGSLHFDPEACSFRERLLEDGYNVYQSEAHGLPLHL
PGNKSPCRDPAPRGPARFLPLPGLPPAPPEPPGILAPQPPDVGSSDPLSMVGGSQGRSPSYES
SEQ ID NO: 10
   >Human mature FGF21 fusion protein with a N-terminal PAS modification (PAS₅₀FGF21) 
SPCASPAAPAPASPAAPAPSAPAAASAAAPAAASAAASAPSAASAAASPAAHPIPDSS
PLLQFGGQVRQRYLYTDDAQQTEAHLEIREDGTVGGAADQSPESLLQLKALKPGVIQILG
VKTSRFLCQRPDGALYGSLHFDPEACSFRERLLEDGYNVYQSEAHGLPLHLPGNKSPHRD
PAPRGPARFLPLPGLPPAPPEPPGILAPQPPDVGSSDPLSMVGGSQGRSPSYES
SEQ ID NO: 11
   > PAS₅₀
   SPASPAAPAPASPAAPAPSAPAAASAAAPAAASAAASAPSAASAAASPAA
SEQ ID NO: 12
   >PAS₅₀ with cysteine inserted at position 3
   SPCASPAAPAPASPAAPAPSAPAAASAAAPAAASAAASAPSAASAAASPAA
SEQ ID NO: 13
   >Human mature FGF21 fusion protein with a N-terminal PAS15 modification (PAS15FGF21) 
SPCASPAAPAPASPAAHPIPDSSPLLQFGGQVRQRYLYTDDAQQTEAHLEIREDGTV
GGAADQSPESLLQLKALKPGVIQILGVKTSRFLCQRPDGALYGSLHFDPEACSFRERLLED
GYNVYQSEAHGLPLHLPGNKSPHRDPAPRGPARFLPLPGLPPAPPEPPGILAPQPPDVGSSD
PLSMVGGSQGRSPSYES
SEQ ID NO: 14
   >Human mature FGF21 fusion protein with a N-terminal PAS30 modification (PAS30FGF21) 
SPCASPAAPAPASPAAPAPSAPAAASAAAPAHPIPDSSPLLQFGGQVRQRYLYTDDA
QQTEAHLEIREDGTVGGAADQSPESLLQLKALKPGVIQILGVKTSRFLCQRPDGALYGSLH
FDPEACSFRERLLEDGYNVYQSEAHGLPLHLPGNKSPHRDPAPRGPARFLPLPGLPPAPPEP
PGILAPQPPDVGSSDPLSMVGGSQGRSPSYES
SEQ ID NO: 15
   >Human mature FGF21 fusion protein with a N-terminal PAS50 modification (PAS50FGF21) 
SPCASPAAPAPASPAAPAPSAPAAASAAAPAAASAAASAPSAASAAASPAAHPIPDSS
PLLQFGGQVRQRYLYTDDAQQTEAHLEIREDGTVGGAADQSPESLLQLKALKPGVIQILG
VKTSRFLCQRPDGALYGSLHFDPEACSFRERLLEDGYNVYQSEAHGLPLHLPGNKSPHRD
PAPRGPARFLPLPGLPPAPPEPPGILAPQPPDVGSSDPLSMVGGSQGRSPSYES
SEQ ID NO: 16
   >Human mature FGF21 fusion protein with a N-terminal PAS100 modification (PAS100FGF21) 
SPCASPAAPAPASPAAPAPSAPAAASAAAPAAASAAASAPSAASAAASPAAPSAPPA
AASPAAPSAPPAAASPAAPAPASPAASAPSAPAAASPAAPAPASHPIPDSSPLLQFGGQVRQ
RYLYTDDAQQTEAHLEIREDGTVGGAADQSPESLLQLKALKPGVIQILGVKTSRFLCQRPD
 
GALYGSLHFDPEACSFRERLLEDGYNVYQSEAHGLPLHLPGNKSPHRDPAPRGPARFLPLP
GLPPAPPEPPGILAPQPPDVGSSDPLSMVGGSQGRSPSYES
SEQ ID NO: 17
   > Human mature FGF21 fusion protein with a N-terminal PAS200 modification (PAS200FGF21)

SPCASPAAPAPASPAAPAPSAPAAASAAAPAAASAAASAPSAASAAASPAAPSAPPA
AASPAAPSAPPAAASPAAPAPASPAASAPSAPAAASPAAPAPASPAAPAPSAPAAASAAAP
AAASAAASAPSASAAASAAAPAAASAAASAPSAASSAASPAAPSAPPAAASPAAPSAPPA
AASPAAPAPASPAAPAPSAPAAAHPIPDSSPLLQFGGQVRQRYLYTDDAQQTEAHLEIRED
GTVGGAADQSPESLLQLKALKPGVIQILGVKTSRFLCQRPDGALYGSLHFDPEACSFRERL
LEDGYNVYQSEAHGLPLHLPGNKSPHRDPAPRGPARFLPLPGLPPAPPEPPGILAPQPPDVG
SSDPLSMVGGSQGRSPSYES

All documents mentioned in the present invention are cited as references in this application, just as each document is individually cited as a reference. In addition, it should be understood that after reading the above teachings of the present invention, a skilled person can make various changes or modifications to the present invention, and these equivalent forms shall also fall within the scope defined by the appended claim set of this application.

## Claims

1. A modified protein, wherein the modified protein includes a protein moiety and a modifying moiety, the protein moiety is optionally linked to PAS at the N-terminus, the modifying moiety is connected directly or via a connector to a cysteine residue in the protein moiety or a cysteine residue in the PAS, and the cysteine residue is either naturally occurring or introduced through mutation.

2. The modified protein of claim 1, wherein the fibroblast growth factor is fibroblast growth factor 21.

3. The modified protein of claim 1, wherein the modifying moiety is an albumin-binding agent.

4. The modified protein of claim 3, wherein the structure of the albumin-binding agent is shown in the following formula:
R₁-(X₁)ₘ-(X₂)ₙ-K(ε-R₂);
wherein K is a lysine;
R₁ is a substituted or unsubstituted C₁₋₂₀ acyl;
m is an integer from 0 to 5;
n is an integer from 0 to 5;
X₁ and X₂ are independently selected from the following group: alanine (Ala), D-alanine (D-Ala), β-alanine (β-Ala), 4-aminobutyric acid (GABA), 2-aminoisobutyric acid (Aib), 2-aminobutyric acid (Abu), arginine (Arg), aspartic acid (Asp), asparagine (Asn), cysteine (Cys), glutamic acid (Glu), D-glutamic acid (D-Glu), γ-glutamic acid (γ-Glu), glutamine (Gln), glycine (Gly), histidine (His), isoleucine (Ile), leucine (Leu), lysine (Lys), proline (Pro), phenylalanine (Phe), serine (Ser), tyrosine (Tyr), threonine (Thr), tryptophan (Trp), valine (Val), methionine (Met), tranexamic acid (Trx), AEEA, PEG;
R₂ is selected from a halogen-substituted C₁₋₆ acyl or H.

5. The modified protein of claim 4, wherein the albumin-binding agent is selected from the group consisting of:

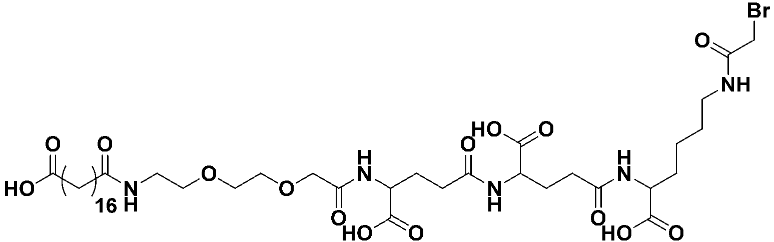

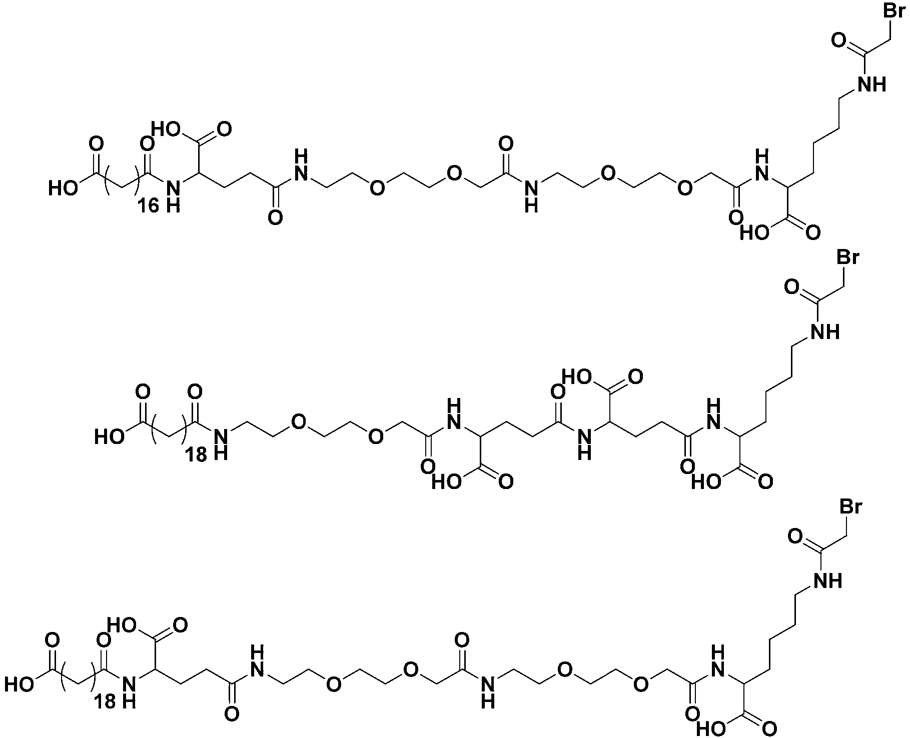

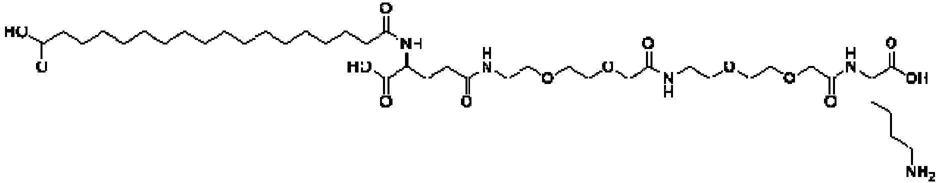

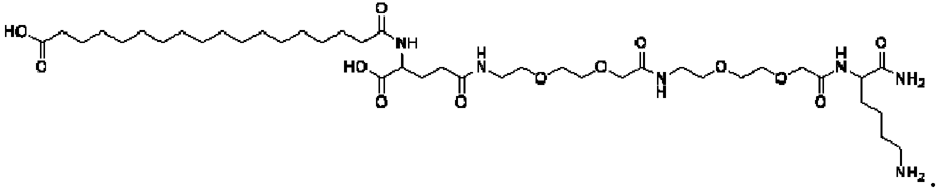

6. The modified protein of claim 1, wherein the PAS is PAS₁₅-PAS₂₀₀; preferably PAS₁₅-PAS₁₀₀; for example, PAS₅₀.

7. The modified protein of claim 1, wherein the connector is a multivalent connector thereby connecting one or more albumin-binding agents and one or more protein moieties.

8. The modified protein of claim 7, wherein the structure of the multivalent connector is shown in the following formula: 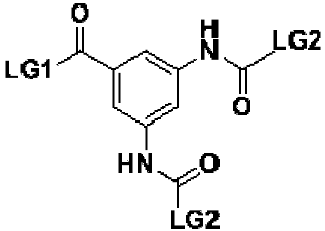
wherein LG1 is a leaving group reactive to primary amino groups; and
LG2 is a leaving group reactive to sulfhydryl groups.

9. The modified protein of any one of claims 1-8, wherein the modified protein is selected from the compounds with the following numbers: 5.1, 5.2, 5.3, 5.4, 5.5, 5.6, 5.7, 5.8, 5.9, 5.10, 5.11, 5.12, 5.13, 5.14, 5.15, 7.2, 7.3, 7.4, 7.5, 12.1, 12.2, 12.3, 12.4, 12.5, 12.6.

10. A protein polymer comprising a multivalent connector and multiple protein monomers, wherein the protein monomers are optionally linked to PAS at their N-terminals, the multivalent connector is connected to the cysteine residues in the protein monomers or the cysteine residues in the PAS, and the cysteine residues are either naturally occurring or introduced through mutation.

11. The protein polymer of claim 10, wherein the PAS is PAS₁₅-PAS₂₀₀; preferably PAS₁₅-PAS₁₀₀; for example, PAS₅₀.

12. The protein polymer of claim 10, wherein the fibroblast growth factor is fibroblast growth factor 21.

13. The protein polymer of claim 10, wherein the structure of the multivalent connector is shown in the following formula:

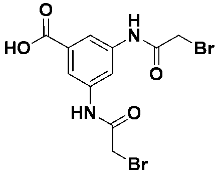

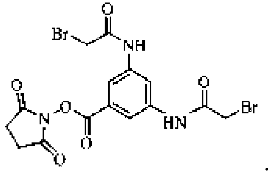

14. The protein polymer of claim 10, wherein the protein polymer is selected from the compounds with the following numbers: 10.1, 10.2, 10.3, 10.4, 10.5.

15. A pharmaceutical composition, comprising the modified protein of any one of claims 1-9 or the protein polymer of any one of claims 10-14, and a pharmaceutically acceptable excipient.

16. Use of the modified protein of any one of claims 1-9 or the protein polymer of any one of claims 10-14 in the preparation of a medicament.

17. An albumin-binding agent, and the structure of the albumin-binding agent is shown in the following formula:
R₁-(X₁)ₘ₋(X₂)ₙ-K(ε-R₂);
wherein K is a lysine;
R₁ is a substituted or unsubstituted C₁₋₂₀ acyl;
m is an integer from 0 to 5;
n is an integer from 0 to 5;
X₁ and X₂ are independently selected from the following group: alanine (Ala), D-alanine (D-Ala), β-alanine (β-Ala), 4-aminobutyric acid (GABA), 2-aminoisobutyric acid (Aib), 2-aminobutyric acid (Abu), arginine (Arg), aspartic acid (Asp), asparagine (Asn), cysteine (Cys), glutamic acid (Glu), D-glutamic acid (D-Glu), γ-glutamic acid (γ-Glu), glutamine (Gln), glycine (Gly), histidine (His), isoleucine (Ile), leucine (Leu), lysine (Lys), proline (Pro), phenylalanine (Phe), serine (Ser), tyrosine (Tyr), threonine (Thr), tryptophan (Trp), valine (Val), methionine (Met), tranexamic acid (Trx), AEEA, PEG;
R₂ is selected from a halogen-substituted C₁₋₆ acyl or H.

18. The albumin-binding agent of claim 17, wherein the albumin-binding agent is selected from the group consisting of:

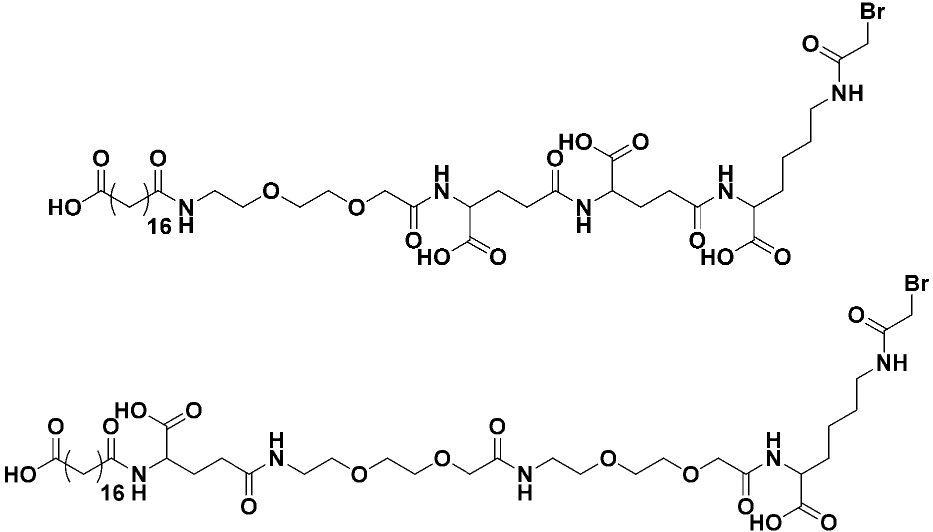

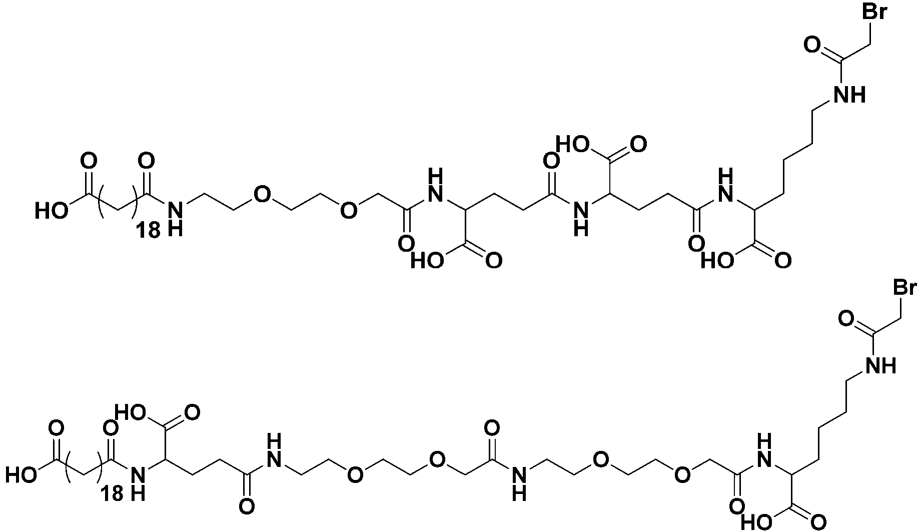

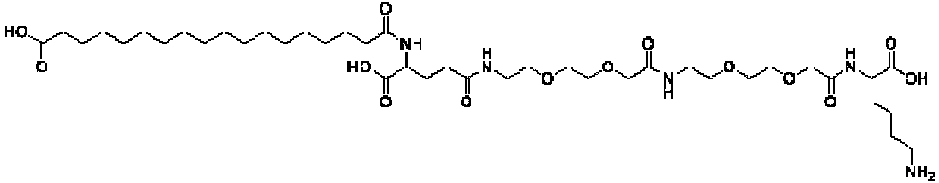

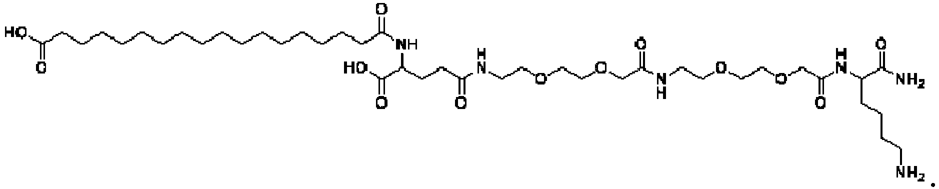

19. A multivalent connector, and the structure of the multivalent connector is shown in the following formula: 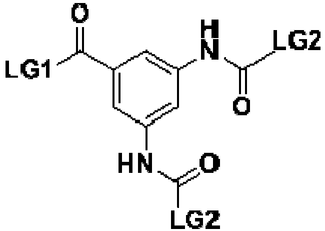
wherein LG1 is a leaving group reactive to primary amino groups; and
LG2 is a leaving group reactive to sulfhydryl groups.

20. A polypeptide, comprising the amino acid sequence shown in SEQ ID NO: 11.

21. A polypeptide, the amino acid sequence of which is shown in SEQ ID NO: 11, but with a cysteine residue inserted at position 3 of the N-terminus.

22. A polypeptide, comprising an amino acid sequence shown in any one of SEQ ID NO: 4-9; preferably an amino acid sequence shown in any one of SEQ ID NO: 5-9.

23. An isolated nucleic acid molecule, encoding the polypeptide of any one of claims 20-22.

24. An expression vector, comprising the isolated nucleic acid molecule of claim 23.

25. A host cell, comprising the expression vector of claim 24, or having the nucleic acid molecule of claim 23 integrated into the genome of the host cell.

26. Use of the polypeptide of any one of claims 20-22 in the preparation of the modified protein of any one of claims 1-9, the protein polymer of any one of claims 10-14, or the pharmaceutical composition of claim 15.
